(19) Europäisches Patentamt
European Patent Office
Office européen des brevets

(11) **EP 3 284 473 A1**

(12) **EUROPEAN PATENT APPLICATION**
published in accordance with Art. 153(4) EPC

(43) Date of publication:
**21.02.2018 Bulletin 2018/08**

(21) Application number: **16780067.1**

(22) Date of filing: **13.04.2016**

(51) Int Cl.:
***A61K 38/00*** *(2006.01)* ***A61K 35/28*** *(2015.01)*
***A61P 7/06*** *(2006.01)* ***A61P 43/00*** *(2006.01)*
***C12N 5/0789*** *(2010.01)*

(86) International application number:
**PCT/JP2016/061889**

(87) International publication number:
**WO 2016/167276 (20.10.2016 Gazette 2016/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **13.04.2015 JP 2015081732**

(71) Applicants:
- **Kyoto University**
  **Kyoto-shi, Kyoto 606-8501 (JP)**
- **Daiichi Sankyo Company, Limited**
  **Tokyo 103-8426 (JP)**

(72) Inventors:
- **HARUYAMA, Munetada**
  **Kobe-shi**
  **Hyogo 650-0047 (JP)**
- **YAMAICHI, Kozo**
  **Kobe-shi**
  **Hyogo 650-0047 (JP)**
- **KITANO, Katsuhiko**
  **Kobe-shi**
  **Hyogo 650-0047 (JP)**
- **NAKAHATA, Tatsutoshi**
  **Kyoto-shi**
  **Kyoto 606-8501 (JP)**

(74) Representative: **Vossius & Partner**
**Patentanwälte Rechtsanwälte mbB**
**Siebertstrasse 3**
**81675 München (DE)**

(54) **AGENT FOR INDUCING AMPLIFICATION OF HEMATOPOIETIC STEM CELLS**

(57) An object of the present invention is to develop a technique for expanding hematopoietic stem cells. Hematopoietic stem cells can be expanded by using at least one HDGF substance.

FIG. 2

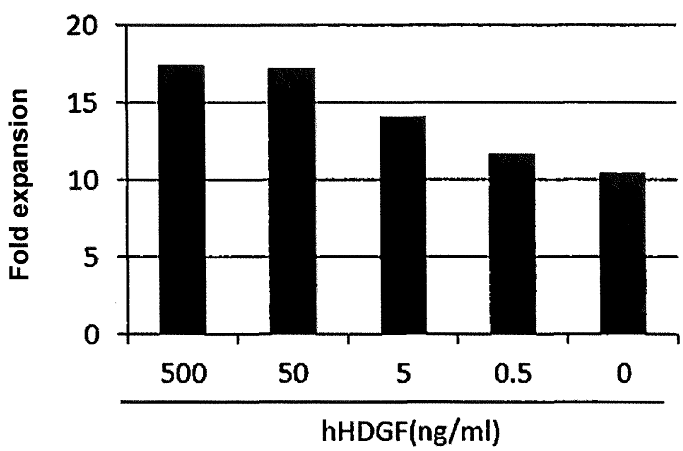


EP 3 284 473 A1

Printed by Jouve, 75001 PARIS (FR)

SEQUENCE LISTING

```
<110>  Kyoto University
       Daiichi Sankyo Company, Limited

<120>  EXPANSION-INDUCER OF HEMATOPOIETIC STEM CELL

<130>  FA1511-16053

<150>  JP 2015-081732
<151>  2015-04-13

<160>  10

<170>  PatentIn version 3.5

<210>  1
<211>  240
<212>  PRT
<213>  Homo sapiens

<400>  1

Met Ser Arg Ser Asn Arg Gln Lys Glu Tyr Lys Cys Gly Asp Leu Val
1               5                   10                  15

Phe Ala Lys Met Lys Gly Tyr Pro His Trp Pro Ala Arg Ile Asp Glu
            20                  25                  30

Met Pro Glu Ala Ala Val Lys Ser Thr Ala Asn Lys Tyr Gln Val Phe
        35                  40                  45

Phe Phe Gly Thr His Glu Thr Ala Phe Leu Gly Pro Lys Asp Leu Phe
    50                  55                  60

Pro Tyr Glu Glu Ser Lys Glu Lys Phe Gly Lys Pro Asn Lys Arg Lys
65                  70                  75                  80

Gly Phe Ser Glu Gly Leu Trp Glu Ile Glu Asn Asn Pro Thr Val Lys
                85                  90                  95

Ala Ser Gly Tyr Gln Ser Ser Gln Lys Lys Ser Cys Val Glu Glu Pro
            100                 105                 110

Glu Pro Glu Pro Glu Ala Ala Glu Gly Asp Gly Asp Lys Lys Gly Asn
        115                 120                 125

Ala Glu Gly Ser Ser Asp Glu Glu Gly Lys Leu Val Ile Asp Glu Pro
    130                 135                 140

Ala Lys Glu Lys Asn Glu Lys Gly Ala Leu Lys Arg Arg Ala Gly Asp
145                 150                 155                 160
```

```
Leu Leu Glu Asp Ser Pro Lys Arg Pro Lys Glu Ala Glu Asn Pro Glu
                165                 170                 175

Gly Glu Glu Lys Glu Ala Ala Thr Leu Glu Val Glu Arg Pro Leu Pro
            180                 185                 190

Met Glu Val Glu Lys Asn Ser Thr Pro Ser Glu Pro Gly Ser Gly Arg
        195                 200                 205

Gly Pro Pro Gln Glu Glu Glu Glu Glu Glu Asp Glu Glu Glu Glu Ala
    210                 215                 220

Thr Lys Glu Asp Ala Glu Ala Pro Gly Ile Arg Asp His Glu Ser Leu
225                 230                 235                 240


<210>  2
<211>  723
<212>  DNA
<213>  Homo sapiens

<400>  2
atgtcgcgat ccaaccggca gaaggagtac aaatgcgggg acctggtgtt cgccaagatg      60

aagggctacc cacactggcc ggcccggatt gacgagatgc ctgaggctgc cgtgaaatca     120

acagccaaca aataccaagt ctttttttc gggacccacg agacggcatt cctgggcccc     180

aaagacctct tcccttacga ggaatccaag gagaagtttg gcaagcccaa caagaggaaa     240

gggttcagcg aggggctgtg ggagatcgag aacaacccta ctgtcaaggc ttccggctat     300

cagtcctccc agaaaaagag ctgtgtggaa gagcctgaac cagagcccga agctgcagag     360

ggtgacggtg ataagaaggg gaatgcagag ggcagcagcg acgaggaagg gaagctggtc     420

attgatgagc cagccaagga gaagaacgag aaaggagcgt tgaagaggag agcaggggac     480

ttgctggagg actctcctaa acgtcccaag gaggcagaaa accctgaagg agaggagaag     540

gaggcagcca ccttggaggt tgagaggccc cttcctatgg aggtggaaaa gaatagcacc     600

ccctctgagc ccggctctgg ccggggggcct ccccaagagg aagaagaaga ggaggatgaa     660

gaggaagagg ctaccaagga agatgctgag gccccaggca tcagagatca tgagagcctg     720

tag                                                                   723


<210>  3
<211>  256
<212>  PRT
<213>  Homo sapiens

<400>  3

Met His Pro Glu Gly Gly Gln Phe Val Pro Gln Leu Leu Gly His Leu
1               5                   10                  15
```

```
Leu Ala Thr Lys Leu Lys Arg Phe Leu Leu Ser Lys Gly Gly Arg Arg
            20                  25                  30

Ala Gln Ile Pro Asp Val Ser Arg Ala Thr Pro His Thr Ile Asp Glu
        35                  40                  45

Met Pro Glu Ala Ala Val Lys Ser Thr Ala Asn Lys Tyr Gln Val Phe
    50                  55                  60

Phe Phe Gly Thr His Glu Thr Ala Phe Leu Gly Pro Lys Asp Leu Phe
65                  70                  75                  80

Pro Tyr Glu Glu Ser Lys Glu Lys Phe Gly Lys Pro Asn Lys Arg Lys
                85                  90                  95

Gly Phe Ser Glu Gly Leu Trp Glu Ile Glu Asn Asn Pro Thr Val Lys
            100                 105                 110

Ala Ser Gly Tyr Gln Ser Ser Gln Lys Lys Ser Cys Val Glu Glu Pro
        115                 120                 125

Glu Pro Glu Pro Glu Ala Ala Glu Gly Asp Gly Asp Lys Lys Gly Asn
    130                 135                 140

Ala Glu Gly Ser Ser Asp Glu Glu Gly Lys Leu Val Ile Asp Glu Pro
145                 150                 155                 160

Ala Lys Glu Lys Asn Glu Lys Gly Ala Leu Lys Arg Arg Ala Gly Asp
                165                 170                 175

Leu Leu Glu Asp Ser Pro Lys Arg Pro Lys Glu Ala Glu Asn Pro Glu
            180                 185                 190

Gly Glu Glu Lys Glu Ala Ala Thr Leu Glu Val Glu Arg Pro Leu Pro
        195                 200                 205

Met Glu Val Glu Lys Asn Ser Thr Pro Ser Glu Pro Gly Ser Gly Arg
    210                 215                 220

Gly Pro Pro Gln Glu Glu Glu Glu Glu Glu Asp Glu Glu Glu Glu Ala
225                 230                 235                 240

Thr Lys Glu Asp Ala Glu Ala Pro Gly Ile Arg Asp His Glu Ser Leu
                245                 250                 255
```

<210> 4
<211> 233

<212> PRT
<213> Homo sapiens

<400> 4

```
Met Glu Gln Arg Ala Gly Gly Asn Arg Val Gln Thr Ser Thr Leu Asn
1               5                   10                  15

Cys Ala Gly Ala Ala Val Ile Asp Glu Met Pro Glu Ala Ala Val Lys
            20                  25                  30

Ser Thr Ala Asn Lys Tyr Gln Val Phe Phe Phe Gly Thr His Glu Thr
        35                  40                  45

Ala Phe Leu Gly Pro Lys Asp Leu Phe Pro Tyr Glu Glu Ser Lys Glu
    50                  55                  60

Lys Phe Gly Lys Pro Asn Lys Arg Lys Gly Phe Ser Glu Gly Leu Trp
65                  70                  75                  80

Glu Ile Glu Asn Asn Pro Thr Val Lys Ala Ser Gly Tyr Gln Ser Ser
                85                  90                  95

Gln Lys Lys Ser Cys Val Glu Glu Pro Glu Pro Glu Pro Glu Ala Ala
            100                 105                 110

Glu Gly Asp Gly Asp Lys Lys Gly Asn Ala Glu Gly Ser Ser Asp Glu
        115                 120                 125

Glu Gly Lys Leu Val Ile Asp Glu Pro Ala Lys Glu Lys Asn Glu Lys
    130                 135                 140

Gly Ala Leu Lys Arg Arg Ala Gly Asp Leu Leu Glu Asp Ser Pro Lys
145                 150                 155                 160

Arg Pro Lys Glu Ala Glu Asn Pro Glu Gly Glu Glu Lys Glu Ala Ala
                165                 170                 175

Thr Leu Glu Val Glu Arg Pro Leu Pro Met Glu Val Glu Lys Asn Ser
            180                 185                 190

Thr Pro Ser Glu Pro Gly Ser Gly Arg Gly Pro Pro Gln Glu Glu Glu
        195                 200                 205

Glu Glu Glu Asp Glu Glu Glu Glu Ala Thr Lys Glu Asp Ala Glu Ala
    210                 215                 220

Pro Gly Ile Arg Asp His Glu Ser Leu
225                 230
```

```
<210>  5
<211>  237
<212>  PRT
<213>  Mus musculus

<400>  5

Met Ser Arg Ser Asn Arg Gln Lys Glu Tyr Lys Cys Gly Asp Leu Val
1               5                   10                  15

Phe Ala Lys Met Lys Gly Tyr Pro His Trp Pro Ala Arg Ile Asp Glu
            20                  25                  30

Met Pro Glu Ala Ala Val Lys Ser Thr Ala Asn Lys Tyr Gln Val Phe
        35                  40                  45

Phe Phe Gly Thr His Glu Thr Ala Phe Leu Gly Pro Lys Asp Leu Phe
    50                  55                  60

Pro Tyr Glu Glu Ser Lys Glu Lys Phe Gly Lys Pro Asn Lys Arg Lys
65                  70                  75                  80

Gly Phe Ser Glu Gly Leu Trp Glu Ile Glu Asn Asn Pro Thr Val Lys
                85                  90                  95

Ala Ser Gly Tyr Gln Ser Ser Gln Lys Lys Ser Cys Ala Ala Glu Pro
            100                 105                 110

Glu Val Glu Pro Glu Ala His Glu Gly Asp Gly Asp Lys Lys Gly Ser
        115                 120                 125

Ala Glu Gly Ser Ser Asp Glu Glu Gly Lys Leu Val Ile Asp Glu Pro
    130                 135                 140

Ala Lys Glu Lys Asn Glu Lys Gly Thr Leu Lys Arg Arg Ala Gly Asp
145                 150                 155                 160

Val Leu Glu Asp Ser Pro Lys Arg Pro Lys Glu Ser Gly Asp His Glu
                165                 170                 175

Glu Glu Asp Lys Glu Ile Ala Ala Leu Glu Gly Glu Arg Pro Leu Pro
            180                 185                 190

Val Glu Val Glu Lys Asn Ser Thr Pro Ser Glu Pro Asp Ser Gly Gln
        195                 200                 205

Gly Pro Pro Ala Glu Glu Glu Glu Gly Glu Glu Glu Ala Ala Lys Glu
    210                 215                 220
```

```
Glu Ala Glu Ala Gln Gly Val Arg Asp His Glu Ser Leu
225                 230                 235


<210>  6
<211>  239
<212>  PRT
<213>  Bos taurus

<400>  6

Met Ser Arg Ser Asn Arg Gln Lys Glu Tyr Lys Cys Gly Asp Leu Val
1               5                   10                  15


Phe Ala Lys Met Lys Gly Tyr Pro His Trp Pro Ala Arg Ile Asp Glu
            20                  25                  30


Met Pro Glu Ala Ala Val Lys Ser Thr Ala Asn Lys Tyr Gln Val Phe
        35                  40                  45


Phe Phe Gly Thr His Glu Thr Ala Phe Leu Gly Pro Lys Asp Leu Phe
    50                  55                  60


Pro Tyr Glu Glu Ser Lys Glu Lys Phe Gly Lys Pro Asn Lys Arg Lys
65                  70                  75                  80


Gly Phe Ser Glu Gly Leu Trp Glu Ile Glu Asn Asn Pro Thr Val Lys
                85                  90                  95


Ala Ser Gly Tyr Gln Ser Ser Gln Lys Lys Ser Cys Val Glu Glu Pro
            100                 105                 110


Glu Pro Glu Pro Glu Ala Thr Glu Gly Asp Gly Asp Lys Lys Gly Asn
        115                 120                 125


Ala Glu Gly Ser Ser Asp Glu Glu Gly Lys Leu Val Ile Asp Glu Pro
    130                 135                 140


Thr Lys Glu Lys Asn Glu Lys Gly Ala Leu Lys Arg Arg Ala Gly Asp
145                 150                 155                 160


Leu Leu Glu Asp Ser Pro Lys Arg Pro Lys Glu Ala Glu Asp Leu Glu
                165                 170                 175


Gly Glu Glu Lys Glu Gly Ala Thr Leu Glu Gly Glu Arg Pro Leu Pro
            180                 185                 190


Val Glu Ala Glu Lys Asn Ser Thr Pro Ser Glu Pro Gly Ser Gly Arg
        195                 200                 205
```

Gly Pro Pro Gln Glu Glu Glu Glu Glu Glu Glu Glu Glu Glu Ala Ala
210 215 220

Lys Glu Asp Ala Glu Ala Pro Gly Leu Arg Asp His Glu Ser Leu
225 230 235

<210> 7
<211> 243
<212> PRT
<213> Sus scrofa

<400> 7

Met Ser Arg Ser Asn Arg Gln Lys Glu Tyr Lys Cys Gly Asp Leu Val
1 5 10 15

Phe Ala Lys Met Lys Gly Tyr Pro His Trp Pro Ala Arg Ile Asp Glu
20 25 30

Met Pro Glu Ala Ala Val Lys Ser Thr Ala Asn Lys Tyr Gln Val Phe
35 40 45

Phe Phe Gly Thr His Glu Thr Ala Phe Leu Gly Pro Lys Asp Leu Phe
50 55 60

Pro Tyr Glu Glu Ser Lys Glu Lys Phe Gly Lys Pro Asn Lys Arg Lys
65 70 75 80

Gly Phe Ser Glu Gly Leu Trp Glu Ile Glu Asn Asn Pro Thr Val Lys
85 90 95

Ala Ser Gly Tyr Gln Ser Ser Gln Lys Lys Ser Cys Val Glu Glu Pro
100 105 110

Glu Pro Lys Ala Asp Ala Ala Glu Gly Asp Gly Asp Lys Lys Gly Asn
115 120 125

Ala Glu Gly Ser Ser Asp Glu Glu Gly Lys Leu Val Ile Asp Glu Pro
130 135 140

Thr Lys Glu Lys Asn Glu Lys Gly Ala Leu Lys Arg Arg Ala Gly Asp
145 150 155 160

Leu Leu Glu Asp Ser Pro Lys Arg Pro Lys Glu Ala Glu Asp Pro Glu
165 170 175

Gly Glu Glu Lys Glu Val Ala Thr Leu Glu Gly Glu Arg Pro Leu Pro
180 185 190

```
Val Glu Ala Glu Lys Asn Ser Thr Pro Ser Glu Pro Ser Ser Gly Arg
        195                 200                 205

Gly Pro Pro Pro Glu Glu Glu Glu Glu Glu Glu Glu Glu Glu Glu Glu
    210                 215                 220

Glu Glu Ala Thr Lys Glu Asp Ala Glu Ala Pro Gly Ile Arg Asp His
225                 230                 235                 240

Glu Ser Leu


<210>  8
<211>  232
<212>  PRT
<213>  Gallus gallus

<400>  8

Met Ser Arg Ser His Arg Gln Lys Glu Tyr Lys Cys Gly Asp Leu Val
1               5                   10                  15

Phe Ala Lys Met Lys Gly Tyr Pro His Trp Pro Ala Arg Ile Asp Glu
            20                  25                  30

Met Pro Glu Ala Ala Val Lys Ser Ser Ser Asn Lys Tyr Gln Val Phe
        35                  40                  45

Phe Phe Gly Thr His Glu Thr Ala Phe Leu Gly Pro Lys Asp Leu Phe
    50                  55                  60

Pro Tyr Glu Glu Cys Lys Glu Lys Phe Gly Lys Pro Asn Lys Arg Lys
65                  70                  75                  80

Gly Phe Ser Glu Gly Leu Trp Glu Ile Glu His Asn Pro Thr Val Lys
                85                  90                  95

Ala Ser Gly Tyr Gln Pro Thr Gln Lys Lys Thr Cys Pro Glu Asp Ala
            100                 105                 110

Glu Pro Glu Gln Glu Pro Glu Gly Asp Gly Glu Lys Lys Gly Asn Ala
        115                 120                 125

Glu Gly Ser Ser Asp Glu Glu Gly Lys Leu Val Ile Asp Glu Gln Ser
    130                 135                 140

Lys Glu Lys Asn Glu Lys Ala Gly Ile Lys Arg Lys Ala Glu Asp Val
145                 150                 155                 160
```

```
Leu Glu Asp Ser Pro Lys Arg Thr Lys Glu Met Glu Gly Gln Glu Ala
                165                 170                 175

Glu Lys Lys Thr Asp Asn Glu Glu Ala Pro Lys Glu Glu Pro Lys Pro
            180                 185                 190

Asn Pro Ala Glu Glu Glu Lys Glu Lys Asn Ser Asp Ala Ala Ser Val
        195                 200                 205

Pro Asp Ala Asn Glu Ala Lys Gln Glu Gly Lys Asp Lys Ala Glu Glu
    210                 215                 220

Val Arg Asp His Lys Glu Ser Leu
225                 230


<210>  9
<211>  304
<212>  PRT
<213>  Capra hircus

<400>  9

Met Ala Pro Pro Ala Phe Gly Ala Pro Leu Pro Ala Asp Glu Ala Tyr
1               5                   10                  15

Leu Arg Pro Gly Met Ser Gly Gly Leu Val Asp Phe Ser Pro Ser Val
            20                  25                  30

Pro Ser Ser Phe Leu His Phe Leu Phe His Ser Pro Arg Lys Pro Ala
        35                  40                  45

Gln Pro Glu Gly Leu Thr His Leu Ser Ser Leu Cys Pro Ser Pro Thr
    50                  55                  60

Pro Arg His His Leu Phe Cys Leu Ser Ser Leu Pro His Phe Pro Gly
65                  70                  75                  80

Pro Asn Cys Leu Leu Pro Gln Ile Phe Gln Phe Leu Gly Gln Ile Asp
                85                  90                  95

Glu Met Pro Glu Ala Ala Val Lys Ser Thr Ala Asn Lys Tyr Gln Val
            100                 105                 110

Phe Phe Phe Gly Thr His Glu Thr Ala Phe Leu Gly Pro Lys Asp Leu
        115                 120                 125

Phe Pro Tyr Glu Glu Ser Lys Glu Lys Phe Gly Lys Pro Asn Lys Arg
    130                 135                 140
```

```
Lys Gly Phe Ser Glu Gly Leu Trp Glu Ile Glu Asn Asn Pro Thr Val
145                 150                 155                 160

Lys Ala Ser Gly Tyr Gln Ser Ser Gln Lys Lys Ser Gly Val Glu Glu
                165                 170                 175

Pro Glu Pro Glu Pro Glu Ala Thr Glu Gly Asp Gly Asp Lys Lys Gly
            180                 185                 190

Asn Ala Glu Gly Ser Ser Asp Glu Glu Gly Lys Leu Val Ile Asp Glu
        195                 200                 205

Pro Ala Lys Glu Lys Asn Glu Lys Gly Ala Leu Lys Arg Arg Ala Gly
    210                 215                 220

Asp Leu Leu Glu Asp Ser Pro Lys Arg Pro Lys Glu Ala Glu Asp Pro
225                 230                 235                 240

Glu Gly Glu Glu Lys Glu Gly Ala Thr Leu Glu Gly Glu Arg Pro Leu
                245                 250                 255

Pro Val Glu Ala Glu Lys Asn Ser Thr Pro Ser Glu Pro Gly Ser Gly
            260                 265                 270

Arg Gly Pro Pro Gln Glu Glu Glu Glu Glu Glu Glu Glu Glu Glu Ala
        275                 280                 285

Ala Lys Glu Asp Ala Glu Ala Pro Gly Leu Arg Asp His Glu Ser Leu
    290                 295                 300


<210>  10
<211>  235
<212>  PRT
<213>  Ovis aries

<400>  10

Met Ala Pro Pro Ala Phe Gly Ala Pro Leu Pro Ala Asp Glu Ala Tyr
1               5                   10                  15

Leu Arg Pro Gly Met Ser Gly Gly Leu Ile Asp Glu Met Pro Glu Ala
            20                  25                  30

Ala Val Lys Ser Thr Ala Asn Lys Tyr Gln Val Phe Phe Phe Gly Thr
        35                  40                  45

His Glu Thr Ala Phe Leu Gly Pro Lys Asp Leu Phe Pro Tyr Glu Glu
    50                  55                  60
```

```
Ser Lys Glu Lys Phe Gly Lys Pro Asn Lys Arg Lys Gly Phe Ser Glu
65                  70                  75                  80

Gly Leu Trp Glu Ile Glu Asn Asn Pro Thr Val Lys Ala Ser Gly Tyr
                85                  90                  95

Gln Gly Ser Gln Lys Lys Ser Ser Val Glu Glu Pro Glu Pro Glu Pro
            100                 105                 110

Glu Ala Thr Glu Gly Asp Gly Asp Lys Lys Gly Asn Ala Glu Gly Ser
        115                 120                 125

Ser Asp Glu Glu Gly Lys Leu Val Ile Asp Glu Pro Ala Lys Glu Lys
    130                 135                 140

Asn Glu Lys Gly Ala Leu Lys Arg Arg Ala Gly Asp Leu Leu Glu Asp
145                 150                 155                 160

Ser Pro Lys Arg Pro Lys Glu Ala Glu Asp Pro Glu Gly Glu Glu Lys
                165                 170                 175

Glu Gly Ala Thr Leu Glu Gly Glu Arg Pro Leu Pro Val Glu Ala Glu
            180                 185                 190

Lys Asn Ser Thr Pro Ser Glu Pro Gly Ser Gly Arg Gly Pro Pro Gln
        195                 200                 205

Glu Glu Glu Glu Glu Glu Glu Glu Glu Glu Ala Ala Lys Glu Asp Ala
    210                 215                 220

Glu Ala Pro Gly Leu Arg Asp His Glu Ser Leu
225                 230                 235
```

## Description

TECHNICAL FIELD

The present invention relates to a technique for expanding hematopoietic stem cells. In particular, this invention relates to a substance for inducing expansion of hematopoietic stem cells, use of said substance, a process for expanding hematopoietic stem cells, and the like.

BACKGROUND ART

Hematopoietic stem cells are cells that are capable of not only differentiating into all types of blood cells but also self-renewing in vivo to provide a lifelong supply of blood cells. Thus, in patients with difficulty in normal blood formation, such as those with leukemia or aplastic anemia, dramatic results have been achieved by a hematopoietic stem cell transplantation therapy in which patients' hematopoietic stem cells are replaced with normal ones. However, there are many unclear points about the mechanism of in vivo expansion of hematopoietic stem cells, and also, hematopoietic stem cells present in tissues collected from donors are difficult to expand in a form capable of withstanding transplantation.

There are three sources of hematopoietic stem cells: bone marrow, peripheral blood, and umbilical cord blood. Transplantations of hematopoietic stem cells coming from these different sources are respectively called "bone marrow transplantation", "peripheral blood stem cell transplantation" and "umbilical cord blood transplantation". Bone marrow transplantation and peripheral blood stem cell transplantation have some problems with a strict match of HLA (human leukocyte antigen) antigens known as major histocompatibility antigens, graft versus host disease (GVHD), and the burden on donors undergoing tissue collection. Umbilical cord blood transplantation has drawbacks in that since the umbilical cord blood contains only limited numbers of hematopoietic stem cells, this technique presents higher rates of engraftment failure than other graft source transplantations and is particularly difficult to apply in adult patients. However, this technique is advantageous in some points, for example: it poses only a small burden on donors since the tissue can be obtained from the umbilical cord at birth; and it does not necessarily require a strict HLA match. It is relatively easy to find donors for umbilical cord blood transplantation since even umbilical cord blood transplants with a match of 4 to 6 of a total of 8 HAL antigens of the four loci (A, B, C, DR) can actually be used for transplantation.

Therefore, if the number of hematopoietic stem cells can be increased in vitro, it will become possible to autologously transplant a small amount of hematopoietic stem cells collected from the bone marrow, peripheral blood or the like of a patient him/herself. It will also become possible to transplant a small amount of hematopoietic stem cells collected from the bone marrow of a donor, thereby achieving a reduction in donor-related risk. Further, increasing the content of hematopoietic stem cells in the umbilical cord blood enables umbilical cord blood transplantation to be applied in adult patients. Thus, if hematopoietic stem cells can be expanded in vitro, it will become possible to solve the current problems with transplantation of hematopoietic stem cells. Accordingly, various studies have been made in hopes of developing an agent or process for expanding hematopoietic stem cells, but there have not been identified any factors that induce in vivo expansion of hematopoietic stem cells.

It has hitherto been known that various attempts have been made to expand hematopoietic stem cells in vitro by culturing hematopoietic stem cells derived from the umbilical cord blood in the presence of a cytokine or a growth factor, such as stem cell factor (hereinafter abbreviated as "SCF"), thrombopoietin (hereinafter abbreviated as "TPO"), FMS-like tyrosine kinase 3 ligand (hereinafter abbreviated as "flt3L"), interleukin-6 (hereinafter abbreviated as "IL-6"), or granulocyte colony stimulating factor (hereafter abbreviated as "GCSF"), but no adequate expansion of hematopoietic stem cells has been achieved. NPTL 1 reports a process for expanding hematopoietic stem cells by adding a low-molecular-weight compound such as copper chelate compound in the presence of different cytokines. This process is mainly intended to expand hematopoietic progenitor cells and contributes to the recovery of hematopoiesis immediately after transplantation, but does not allow expansion of hematopoietic stem cells capable of long-term production of blood cells. NPTL 2 reports that other low-molecular-weight compounds, *i.e.,* hydrocarbon antagonists, promote the expansion of hematopoietic stem cells capable of withstanding secondary transplantation, in the presence of different cytokines. However, the culture period required for cell expansion is as long as 21 days, and no data has presently been presented on repopulation of the bone marrow after secondary transplantation; thus, it is uncertain whether hematopoietic stem cells are capable of maintaining their hematopoietic ability for a long time. Further, since this phenomenon is triggered by addition of a compound not present in the body, it is questionable whether this phenomenon simulates in vivo expansion of hematopoietic stem cells.

There is also reported a process for hematopoietic stem cell expansion in a co-culture system with hematopoiesis-supporting cells (NPTL 3). However, there are some problems, such as unidentified effects of an unknown substance/factor derived from the supporting cells, and risk of inclusion of the supporting cells during transplantation.

Further, G-CSF treatment is known as a process intended for in vivo recovery of blood cells. However, this treatment is effective only in granulocytes and is not capable of promoting recovery of all types of blood cells, including platelets and erythrocytes.

Hepatoma-derived growth factor (hereinafter abbreviated as "HDGF") is a protein purified from a culture supernatant of the human hepatoma-derived cell line, HuH-7, using mouse Swiss 3T3 cell proliferation as an indicator (NPTL 4). HDGF is reported to be an acidic protein consisting of 240 amino acids and containing two N-glycosylation sites in the molecule. Since HDGF is present in a supernatant of cultured cells but does not have a signal sequence required for secretion in its N-terminal sequence, it is presumed that this factor is secreted via a secretory signal-independent pathway.

To date, the cDNA of HDGF has been cloned not only from human sources but also mouse, rat and bovine sources. Human HDGF and mouse HDGF have 88% homology not only at the genetic level but also at the amino acid level, and are well conserved between species. In particular, human HDGF and mouse HDGF are in complete agreement with each other in terms of the amino acids of the HATH region essential for intracellular mitigation (NPTL 5). As seen above, the HDGFs of higher vertebrate animals have highly homologous amino acid sequences even in the case where these animals are of phylogenetically distant species. Also, it is known that there are several types of HDGF isoforms, which are at least 90% homologous to each other. Further, HDGF-encoding genes were identified in many biological species by genome sequence analysis.

It has hitherto been reported that HDGF has proliferative activity on fibroblasts, vascular endothelial cells, smooth myocytes, and embryonic hepatocytes (NPTL 6), but there has been no report on the effects of HDGF on expansion of hematopoietic stem cells or hematopoietic progenitor cells.

## CITATION LIST

### NON-PATENT LITERATURES

NPTL 1: Peled T., Gluckman E., Hasson N., Adi S., Assor H., Yudin D., et al., Chelatable cellular copper modulates differentiation and self-renewal of cord blood-derived hematopoietic progenitor cells. Exp Hematol 2005; 33: 1092-1100.

NPTL 2: Boitano A.E., Wang J., Romeo R., et al., Aryl hydrocarbon receptor antagonists promote the expansion of human hematopoietic stem cells. Science. 2010; 329 (5997): 1345-1348.

NPTL 3: Amsellem S., Pflumio F., Bardinet D., Izac B., Charneau P., Romeo P.H., et al., In vitro expansion of human hematopoietic stem cells by direct delivery of the HOXB4 homeoprotein. Nat Med. 2003; 9: 1423-7.

NPTL 4: Nakamura H., Izumoto Y., Kambe H., Kuroda T., Mori T., Kawamura K., Yamamoto H., and Kishimoto T. (1994) J. Biol. Chem. 269, 25143-25149

NPTL 5: Izumoto Y., Kuroda T., Harada H., Kishimoto T., Nakamura H., Hepatoma-derived growth factor belongs to a gene family in mice showing significant homology in the amino terminus. (1997) Biochem Bioplays Res Commun. 238(1): 26-32.

NPTL 6: Enomoto H., Yoshida K., Kishima Y., Kinoshita T., Yamamoto M., Everett A.D., Miyajima A., Nakamura H., Hepatoma-derived growth factor is highly expressed in developing liver and promotes fetal hepatocyte proliferation. (2002) Hepatology. 36(6): 1519-27


## SUMMARY OF INVENTION

### TECHNICAL PROBLEM

An object to be achieved by the present invention is to develop a technique for expanding hematopoietic stem cells. In particular, an object of this invention is to find a biologic factor capable of inducing expansion of hematopoietic stem cells and induce in vivo and in vitro expansion of hematopoietic stem cells using said factor.

### SOLUTION TO PROBLEM

The present inventors have conducted a vigorous search for a biologic factor capable of inducing expansion of human hematopoietic stem cells, and as a result, found that HDGF is capable of inducing expansion of hematopoietic stem cells, that when such expanded cells were transplanted into mice, those cells differentiated into all types of blood cells, and that such expanded cells were successfully engrafted and expanded even after secondary transplantation into mice. After further studies, the inventors have completed this invention.

More specifically, the present invention includes the following.

(1) An expansion inducer for hematopoietic stem cells, comprising at least one HDGF substance.
(2) The expansion inducer as set forth in (1), wherein the HDGF substance is selected from:

(a) a polypeptide comprising the amino acid sequence of positions 30 to 208 of SEQ ID NO:1;
(b) a polypeptide consisting of an amino acid sequence having at least 60% amino acid sequence similarity to the amino acid sequence of SEQ ID NO:1;
(c) a polypeptide consisting of an amino acid sequence derived from the amino acid sequence of SEQ ID NO:1 by deletion, substitution and/or addition of one or several amino acids at one or several positions; or
(d) a polypeptide comprising an amino acid sequence encoded by a nucleotide sequence capable of hybridizing under stringent conditions to a polynucleotide comprising a nucleotide sequence complementary to the nucleotide sequence of SEQ ID NO:2.

(3) The expansion inducer as set forth in (1), wherein the HDGF substance comprises a polypeptide consisting of the amino acid sequence of any of SEQ ID NOs:1 and 3 to 10.
(4) The expansion inducer as set forth in (1), wherein the HDGF substance is a polypeptide consisting of the amino acid sequence of any of SEQ ID NOs:1 and 3 to 10.
(5) The expansion inducer as set forth in any of (1) to (4), further comprising at least one cytokine selected from the group consisting of stem cell factors (SCF), flk-2/flt-3 ligand (FL), thrombopoietin (TPO), granulocyte-macrophage colony-stimulating factors (GM-CSF) and IL-6.
(6) The expansion inducer as set forth in (5), comprising at least thrombopoietin.
(7) The expansion inducer as set forth in any of (1) to (6), wherein the expansion inducer is administered to a subject for the purpose of treatment of a disease caused by depressed or impaired hematopoietic function.
(8) The expansion inducer as set forth in any of (1) to (6), wherein the expansion inducer is used to prepare a biological material for transplantation therapy in a subject with depressed or impaired hematopoietic function.
(9) The expansion inducer as set forth in (8), wherein the expansion inducer is used in a reagent, a culture medium, or a culture substrate.
(10) A process for expanding hematopoietic stem cells, the process comprising the step of contacting the expansion inducer as set forth in any of (1) to (6) in vitro with a sample comprising hematopoietic stem cells.
(11) The process as set forth in (10), wherein the sample comprising hematopoietic stem cells is a sample derived from the bone marrow, peripheral blood, or umbilical cord blood collected from a living organism.
(12) A process for producing a biological material comprising hematopoietic stem cells, the process comprising the step of expanding hematopoietic stem cells in vitro using the process as set forth in (10) or (11).
(13) A biological material comprising hematopoietic stem cells, the biological material produced by the process as set forth in (12).
(14) The biological material as set forth in (13), wherein the biological material is intended for use in transplantation therapy.
(15) A process for producing a biological material comprising mature blood cells, the process comprising the steps of: contacting the expansion inducer as set forth in any of (1) to (6) in vitro with a sample comprising hematopoietic stem cells to expand the hematopoietic stem cells; and inducing the expanded hematopoietic stem cells to differentiate into mature blood cells.
(16) A biological material comprising mature blood cells, the biological material produced by the process as set forth in (15).
(17) The biological material as set forth in (16), wherein the biological material is intended for use in transplantation medicine.
(18) A process for producing a biological material comprising hematopoietic stem cells, the process comprising the steps of: contacting the expansion inducer as set forth in any of (1) to (6) in vitro with a sample comprising hematopoietic stem cells to expand the hematopoietic stem cells; and introducing a gene for gene therapy into the hematopoietic stem cells before or after the contact with the expansion inducer.
(19) The process as set forth in (18), wherein the gene for gene therapy is selected from the group consisting of: the ADA gene for treatment of adenosine deaminase deficiency, the γc chain gene for treatment of X-linked severe combined immunodeficiency disease, the gp91-phox gene for treatment of chronic granulomatous disease, the glucocerebrosidase gene for treatment of Gaucher's disease, and a factor VIII or IX gene for treatment of hemophilia.
(20) A biological material comprising hematopoietic stem cells, the biological material produced by the process as set forth in (18) or (19).

ADVANTAGEOUS EFFECTS OF INVENTION

According to the present invention, hematopoietic stem cells can be expanded in vitro and in vivo by using a HDGF substance.

When the present invention is applied in vitro, long-term hematopoietic stem cells having long-term bone marrow repopulating ability can be expanded, and thus, the expanded cells can be used as cells for transplantation of hematopoietic stem cells. In particular, such an expansion effect is observed on hematopoietic stem cells in umbilical cord blood, and it is expected that this invention will enable umbilical cord blood transplantation in adults. The hematopoietic stem cells expanded in this invention maintain their ability to differentiate into different types of blood cells and thus are useful as a material for transplantation of particular types of blood cells. The cell transplantation can be performed in a highly safe manner, since HDGF prepared aseptically and at a low-impurity level of quality in a controlled environment can be used in the process of cell expansion. Also, hematopoietic stem cells and/or hematopoietic progenitor cells can be expanded in a shorter time than in the case of using conventionally known processes.

Since hematopoietic stem cells can be expanded according to the present invention, hematopoietic progenitor cells and particular types of blood cells, such as erythrocytes and platelets, can also be produced in large amounts.

Further, production of all types of blood cells is promoted by increasing the amount of HDGF in the living body through administration of an agent comprising HDGF as an active component or through enhancement of HDGF gene expression; therefore, it is expected that patients in need of hematopoietic stem cell transplantation can be treated with medication. Furthermore, such an agent is applicable for various purposes including recovery of blood cells after administration of an anticancer drug, and is not only capable of preventing organ failures associated with anaemia, infections, or thrombocytopenia but is also particularly expected to eliminate the need for platelet transfusion or the like which have problems with ease of access, expiration date, infections (with bacteria, protozoans, viruses, etc.), and immune-related side effects including anaphylaxis.

Hematopoietic stem cells have pluripotency to differentiate into all types of blood cells, and are also capable of self-renewing in vivo. Since all hematopoietic progenitor cells and mature blood cells, including autologous cells, are formed from one cell, it is considered that there exists an ingenious and complicated control mechanism that prevents hematopoietic stem cells from becoming genetically mutated and cancerous at the time of their differentiation and proliferation. It is also considered that the control mechanism can address urgent conditions such as inflammation. For this reason, there has conventionally been proposed a process for applying stimulus using a compound not present in the body, but there was concern that the stimulus applied by this process may be a stimulus that cannot be addressed in the body, and may disturb the ingenious control mechanism of hematopoietic stem cells, resulting in cell cancerization or the like. On the other hand, HDGF is a factor present in the body, and the hematopoietic stem cell expansion effect of HDGF is considered as an appropriate biological effect. No abnormal cell expansion caused by using HDGF was observed, as described herein. Thus, the expansion inducer of the present invention is presumed to be capable of expanding hematopoietic stem cells without disturbing the control mechanism of the hematopoietic stem cells.

BRIEF DESCRIPTION OF DRAWINGS

[FIG. 1] FIG. 1 shows the amino acid sequence of hHDGFa and the nucleotide sequence of cDNA encoding said amino acid sequence.

[FIG. 2] FIG. 2 shows the dose-dependent activity of hHDGF on expansion of hCD34+ cells. The x-axis represents hHDGF concentrations (ng/mL), and the y-axis represents fold expansions calculated based on the initial number of hCD34+ cells ($1\times10^4$ cells/mL).

[FIG. 3] FIG. 3 is a graph showing the activities of HDGF and different cytokines used in an hCD34+ cell expansion assay. In the graph, the x-axis represents cytokines and the like added. H represents hHDGF, T represents hTPO, S represents hSCF, F represents hFlt3-L, and different combinations of any of these symbols represent concurrent administration of the corresponding cytokines (*cf.,* "None": no addition). The y-axis represents fold expansions calculated after 7 days of culture based on the initial number of hCD34+ cells (number of cells seeded: $1\times10^4$ cells/mL).

[FIG. 4] FIG. 4 shows the results of the FACS analysis of CD34+ cells uncultured (UC) and cultured in the presence of hHDGF (H) and hTPO (T) (A: untreated hCD34+ cells; B: hCD34+ cells cultured in the presence of hHDGF for 3 days; C: hCD34+ cells cultured in the presence of hTPO for 5 days; and D: hCD34+ cells cultured in the presence of both hHDGF and hTPO for 5 days). The x-axis represents hCD34 expression levels (PB-CD34-A), and the y-axis represents hCD38 expression levels (FITC-CD38-A).

[FIG. 5] FIG. 5 shows the results of the FACS analysis of hCD34 (x-axis: APC-CD34-A) and hCD38 (y-axis: PE-CD38-A) in the bone marrow samples at 23 weeks after the transplantation performed in the transplantation study of hCD34+ cells treated under different conditions into NOG mice -- the results are arranged in tandem for each individual. The different columns in order from left to red show the results for the following groups: column A: UC group (uncultured group); column B: H group (a group cultured in the presence of hHDGF alone for 3 days); column C: T group (a group cultured in the presence of hTPO alone for 5 days); and column D: HT group (a group cultured in the presence of hHDGF+hTPO for 5 days). The number of individuals in each group was 5, but the number of individuals in the H group was 4 because one mouse died during the study.

[FIG. 6] FIG. 6 is a graph showing the averaged values (y-axis) of the absolute numbers of hCD34+hCD38- cells in the bone marrow samples at 23 weeks after the transplantation performed in the transplantation study of hCD34+ cells treated under different conditions into NOG mice. In the x-axis, UC represents an uncultured group; H represents a group cultured in the presence of hHDGF alone for 3 days; T represents a group cultured in the presence of hTPO alone for 5 days; and HT represents a group cultured in the presence of hHDGF+hTPO for 5 days.

[FIG. 7] FIG. 7 shows the results of the FACS analysis of hCD34+ cells uncultured (UC) and cultured in the presence of hHDGF and hTPO (HT) (A: untreated CD34+ cells; and B: CD34+ cells cultured in the presence of both hHDGF and hTPO for 5 days). The x-axis represents CD34 expression levels (PB-CD34-A), and the y-axis represents CD38 expression levels (FITC-CD38-A).

[FIG. 8] FIG. 8 shows the results of the FACS analysis conducted on different blood cells using cell surface markers to analyze their pluripotency at 24 weeks after the primary transplantation performed in the transplantation study of hCD34+ cells treated under different conditions into NOG mice (UC: uncultured group; and HT: hHDGF/hTPO-cultured group). The y-axis represents the expression level of an intended antigen marker, and the x-axis represents the expression level of a marker of interest. CD45 (y-axis: FITC-CD45-A; x-axis: PB-hCD45-A), CD33 (y-axis: PE-Cy5-CD19-A; x-axis: PE-CD33-A), CD19•CD3 (y-axis: PE-Cy5-CD19-A; x-axis: APC-CD3-A), CD235a (y-axis: PB-hCD45-A; x-axis: PE-CD235a-A), Erythrocyte (y-axis: APC-TER119-A; x-axis: FITC-CD235a-A), Platelet (y-axis: SSC-A; x-axis: APC-CD41-A).

[FIG. 9] FIG. 9 shows the results of the FACS analysis of hCD34 (x-axis: APC-CD34-A) and hCD38 (y-axis: PE-CD38-A) in the bone marrow samples collected from individual mice at 24 weeks after the primary transplantation performed in the transplantation study of hCD34+ cells treated under different conditions into NOG mice. UC represents an uncultured group (n=5), and HT represents a hHDGF/hTPO-cultured group (n=8).

[FIG. 10] FIG. 10 shows the results of the FACS analysis conducted on different human blood cells using cell surface markers to analyze their pluripotency at 20 weeks after the secondary transplantation performed in the transplantation study of hCD34+ cells treated under different conditions into NOG mice (UC: uncultured group; and HT: hHDGF/hTPO-cultured group). The y-axis represents the expression level of an intended cell surface marker, and the x-axis represents the expression level of a marker of interest. CD45 (y-axis: FITC-mCD45-A; x-axis: PB-hCD45-A), CD19•CD33 (y-axis: PE-Cy5-CD19-A; x-axis: PE-CD33-A), CD3 (y-axis: PE-Cy5-CD19-A; x-axis: APC-CD3-A), CD235a (y-axis: PB-hCD45-A; x-axis: PE-CD235a-A), Erythrocyte (y-axis: APC-TER119-A; x-axis: FITC-CD235a-A), Platelet (y-axis: SSC-A; x-axis: APC-hCD41-A).

[FIG. 11] FIG. 11 shows the results of the FACS analysis of hCD34 (x-axis: APC-CD34-A) and hCD38 (y-axis: PE-CD38-A) in the bone marrow samples collected from individual mice at 20 weeks after the secondary transplantation performed in the transplantation study of hCD34+ cells treated under different conditions into NOG mice. UC represents an uncultured group (n=5), and HT represents a hHDGF/hTPO-cultured group (n=8).

[FIG. 12] FIG. 12 is a graph showing the averaged values (y-axis) of the percentages of hCD45+ cells relative to all leukocytes (hCD45+ cells plus mCD45+ cells) in peripheral blood samples at 4 weeks after the transplantation conducted by administration of different concentrations of HDGF to NOG mice transplanted with human umbilical blood-derived CD34+ cells.

[FIG. 13] FIG. 13 shows the regions of different truncated fragments of hHDGFa in the sequence of hHDGFa.

[FIG. 14] FIG. 14 is a set of graphs showing the total number of cells and the absolute number of CD34+CD38- cells, which were both calculated in CD34+ cells cultured in the presence of different truncated fragments of hHDGFa for 5 days. UN represents an uncultured group; TPO represents a hTPO-treated group; HT represents a full-length hHDGFa+hTPO-treated group; H1-208+T represents an H1-208+hTPO-treated group; H30-240+T represents an H30-240+hTPO-treated group; H30-208+T represents an H30-208+hTPO-treated group; H4-240+T represents an H4-240+hTPO-treated group; H1-230+T represents an H1-230+hTPO-treated group; and H1-150+T represents an H1-150+hTPO-treated group.

## DESCRIPTION OF EMBODIMENTS

In the present invention, hematopoietic stem cells are expanded using at least one HDGF substance. In one aspect, this invention provides an expansion inducer for hematopoietic stem cells, comprising at least one HDGF substance. The expansion inducer of this invention is provided as a HDGF substance, or an agent, reagent or the like comprising said HDGF substance, which are intended for use to expand hematopoietic stem cells. When used in vitro, the expansion inducer of this invention is provided in the form of a reagent, additive, culture medium, culture substrate or the like comprising a HDGF substance, which are intended for use in culturing a sample containing hematopoietic stem cells. When applied in vivo, the expansion inducer of this invention is provided in the form of a medicament comprising a HDGF substance as an active component, which is intended for use in the treatment of a disease associated with depressed or impaired hematopoietic function.

Further, the expansion inducer of the present invention may be provided as a reagent for study of hematopoiesis-related cells. For example, a factor that regulates differentiation and proliferation of hematopoietic stem cells or hematopoietic progenitor cells can be elucidated by analyzing the type of colony forming cells and changes in cell surface differentiation markers and expression genes during culture under the coexistence of hematopoietic stem cells or hematopoietic progenitor cells with a factor of interest.

<Hematopoietic stem cells>

The "hematopoietic stem cells" refers to cells that have pluripotency to differentiate into all types of blood cells and which are capable of self-renewing. As referred to above, "self-renewing" means that a stem cell produces a cell having the same function and properties as said stem cell through cell division. In other words, cells produced by self-renewal of hematopoietic stem cells have pluripotency to differentiate into all types of blood cells and self-renewal ability. Because of having these functions, hematopoietic stem cells are capable of reconstructing the bone marrow and continuously producing all types of blood cells, when transplanted or otherwise introduced to an animal with destroyed bone marrow.

Differentiation of hematopoietic stem cells means that long-term hematopoietic stem cells convert into short-term hematopoietic stem cells, short-term hematopoietic stem cells convert into pluripotent hematopoietic progenitor cells, pluripotent hematopoietic progenitor cells convert into unipotent hematopoietic progenitor cells, and unipotent hematopoietic progenitor cells convert into cells having a unique function, *i.e.,* mature blood cells such as erythrocytes, leukocytes and megakaryocytes. It is said that among these series of cells related to differentiation of hematopoietic stem cells, the only cells having self-renewal ability are long-term and short-term hematopoietic stem cells, and those cells downstream of pluripotent hematopoietic progenitor cells have cell division ability associated with differentiation but do not have self-renewal ability. In the field of transplantation therapy and the like, it is important that transplanted cells function continuously in the body of transplantation recipients, and for that purpose, it is important to transplant cells containing large amounts of hematopoietic stem cells having self-renewal ability.

It is known that the expression pattern of particular cell surface antigens changes in the process of differentiation of hematopoietic stem cells. The "CD34+ cells" refers to cells expressing the CD (cluster of differentiation) 34 antigen on the cell surface. This antigen is a marker for hematopoietic stem cells and hematopoietic progenitor cells, and its expression decreases with cell differentiation. In other words, unless otherwise defined, the "CD34+ cells" refers to a cell population containing hematopoietic stem cells and hematopoietic progenitor cells. The "CD38-" means that no CD38 antigen is expressed on the cell surface. Since the expression of this antigen increases with differentiation of hematopoietic stem cells, this antigen is generally not expressed in undifferentiated hematopoietic stem cells.

The "CD34+CD38- cells" refers to a cell population expressing the CD34 antigen, but not the CD38 antigen, on the cell surface. CD34+CD38- cells are a cell population with higher content of hematopoietic stem cells than CD34+ cells.

There exists a hierarchy of hematopoietic stem cells, and these stem cells are classified into short-term hematopoietic stem cells and long-term hematopoietic stem cells depending on their hematopoiesis-sustaining ability. Short-term hematopoietic stem cells are capable of repopulating the bone marrow in vitro or during primary transplantation, but are unable to maintain this ability during secondary transplantation. On the other hand, long-term hematopoietic stem cells have an ability to repopulate the bone marrow even during secondary transplantation (long-term bone marrow repopulating ability). In the present invention, unless otherwise specified, the "hematopoietic stem cells" refers to a cell population containing short-term hematopoietic stem cells and/or long-term hematopoietic stem cells.

In the present invention, the "expansion" of hematopoietic stem cells generally means that when a cell population containing hematopoietic stem cells is subjected to a certain treatment in vitro, the number of hematopoietic stem cells contained in the cell population increases. The expansion of hematopoietic stem cells can be achieved by, for example, inducing hematopoietic stem cells having the same properties to self-renew from at least part of the hematopoietic stem cells contained in the cell population. Hematopoietic stem cells are known to be capable of self-renewing in vivo, but no particular factor that induces the self-renewal of these cells has not been identified -- thus, it has been difficult to expand hematopoietic stem cells in vitro by inducing the self-renewal of hematopoietic stem cells in the same manner as in an in vivo environment. Since hematopoietic stem cells convert into downstream cells as differentiation progresses, allogeneic cells will decrease or disappear if they are not expanded through self-renewal. However, the use of the expansion inducer of this invention makes it possible to expand hematopoietic stem cells, so that the number of hematopoietic stem cells can be maintained or increased in vitro above a certain level.

The self-renewal of hematopoietic stem cells can be determined using an increase in the number of CD34+CD38- cells as an indicator. In vivo self-renewal can be confirmed by transplanting hematopoietic stem cells in an animal and measuring the number of transplantation-derived CD34+CD38- cells present in the bone marrow collected from the animal after a certain period of time.

In an in vitro setting, if the number of CD34+CD38- cells after a certain treatment increases as compared with that number before the certain treatment, it can be considered that the self-renewal of hematopoietic stem cells has been induced. Further, the pluripotency of hematopoietic stem cells can be investigated by a known method, or the functionality of expanded cells functioning as hematopoietic stem cells can be confirmed by repeatedly performing the same treatment on at least part of the resulting cells and determining changes in the number of CD34+CD38- cells.

The differentiation pluripotency of hematopoietic stem cells can be determined by subjecting the resulting cells (*i.e.,* CD34+ cell population or CD34+CD38- cell population) to a treatment for inducing differentiation into at least two different types of hematopoietic progenitor cells or blood cells derived therefrom, and observing the differentiation into desired hematopoietic progenitor cells or blood cells.

The functionality of human cells as hematopoietic stem cells is commonly confirmed using a technique of transplantation into an immunodeficient mouse. The Central Institute for Experimental Animals in Japan recently developed an immunodeficient mouse which is capable of producing almost all types of human mature blood cells when transplanted with human hematopoietic stem cells (NOD.Cg-$Prkdc^{scid}$ $Il2rg^{tmlSug}$/Jic; hereinafter referred to as "NOG mouse"; Blood (2012), 100(9): 113-1124). By transplanting cells or a tissue of interest in this mouse, the transplanted cells can be determined for their ability to migrate and engraft into the bone marrow, their pluripotency, and their bone marrow repopulating ability. Further, by collecting a bone marrow sample from an immunodeficient mouse in which transplantation has once been successful and transplanting the collected sample into another NOG mouse again (secondary transplantation), the transplanted cells can be determined for their self-renewal ability and long-term bone marrow repopulating ability. The cells/tissue whose functionality as hematopoietic stem cells is confirmed by this technique can be regarded as cells/tissue suitable for use in actual hematopoietic stem cell transplantation.

There are two types of hematopoietic progenitor cells: pluripotent hematopoietic progenitor cells and unipotent hematopoietic progenitor cells. Pluripotent hematopoietic progenitor cells have pluripotency to differentiate into all types of blood cells but are unable to self-renew. Accordingly, pluripotent hematopoietic progenitor cells can produce blood cells temporarily, but once all the cells differentiate, depletion of hematopoietic progenitor cells occurs so that destroyed bone marrow can no longer be repopulated.

Unipotent hematopoietic progenitor cells are capable of differentiating into one or several particular types of blood cells but do not have self-renewal ability. For example, there are various types of unipotent hematopoietic progenitor cells, including granulocyte-macrophage colony forming units (CFU-GM), erythrocytic progenitor cells such as erythrocyte burst-forming units (BFU-E), and mixed colony-forming units (CFU-Mix).

As a hematopoietic progenitor cell assay by a functional method, the colony formation method (*e.g.,* Ueda T., et al., J. Clin. Invest. (2000) 105: 1013-1021) has traditionally been used. The most immature colony determined by the colony formation method is a mixed colony in which erythrocytes and leukocytes are mixed (hereinafter abbreviated as "CFU-Mix"). Since hematopoietic progenitor cells have no self-renewal activity, if an increased number of hematopoietic progenitor cells -- *e.g.,* an increased number of CFU-Mix relative to the control group -- is observed by the colony formation method, then it is suggested that hematopoietic stem cells are activated to increase the number of hematopoietic progenitor cells.

In the present invention, the "mature blood cells" refers to a mature cell population formed from hematopoietic stem cells through hematopoietic progenitor cells. Examples of the mature blood cells include erythrocytes, neutrophils, monocytes, acidophils, basophils, macrophages, platelets, mast cells, T-cells, B-cells, NK cells, and NKT cells. Mature blood cells can be obtained by inducing differentiation of hematopoietic stem cells, or hematopoietic progenitor cells situated upstream of such mature blood cells. These mature blood cells can be distinguished by a known method using a specific marker expressed in the cells. There are various known markers, including CD33 used as a marker for monocytes, macrophages and granulocytes, CD41 used as a marker for megakaryocytes and platelets, CD235a as an erythrocyte marker, CD19 used as a B-cell marker, and CD3 used as a T-cell marker.

<HDGF substance>

In the present invention, the "HDGF substance" refers to a substance that comprises a polypeptide having sequence similarity above a certain level to the amino acid sequence of human HDGFa (hHDGFa) as represented in SEQ ID NO:1 (hereinafter referred to as "HDGF peptide") and which has an activity to induce expansion of hematopoietic stem cells. The sequence similarity of a HDGF peptide in the HDGF substance to the amino acid sequence of HDGFa is preferably at least 60%, more preferably at least 70%, still more preferably at least 80%, yet more preferably at least 90%, most preferably at least 95%. The HDGF peptide includes not only hHDGFa but also an analogue thereof, *i.e.,* a polypeptide having an activity to induce expansion of hematopoietic stem cells. The HDGF substance includes a HDGF peptide *per se,* a modified form thereof, and the like.

As referred to above, "hHDGFa" is a protein of 240 amino acids (the amino acid sequence is shown in SEQ ID NO:1, and the nucleotide sequence of cDNA encoding the amino acid sequence is shown in SEQ ID NO:2), which is known to have heparin-binding ability and an activity to stimulate the expansion of a wide variety of cells, such as fibroblasts, vascular endothelial cells, smooth myocytes, and embryonic hepatocytes. In the amino acid sequence of this protein, the HATH (homologous to amino-terminus HDGF) domain, a region of positions 1 to 100 toward the N-terminus, is believed to be responsible for heparin binding, and is conserved in all HRP (HDGF-related protein) family proteins. The HATH domain contains a PWWP motif (positions 24 to 27) and a NLS (nuclear localization signal) sequence (positions 75 to 80). In the amino acid sequence of SEQ ID NO:1, the region of positions 155 to 170 is called a bipartite NLS sequence. The PWWP motif is a structure commonly found in nuclear proteins responsible for differentiation and proliferation, and is believed to be involved in protein-protein interaction. The NLS sequence and the bipartite NLS sequence are believed to be involved in nuclear localization of proteins. Aside from hHDGFa, there are two other isoforms of naturally occurring hHDGF (hHDGFb (SEQ ID NO:3) and hHDGFc (SEQ ID NO:4)). In SEQ ID NO:1, the region of positions 1 to 29 is a sequence specific to hHDGFa, and the region of positions 30 to 240 is a sequence that can be found in common among all isoforms. Namely, in the amino acid sequence of SEQ ID NO:1, the region of positions 30 to 100 in the HATH domain is a particularly conserved sequence.

In the present invention, the analogue of hHDGFa can be exemplified by: (a) a polypeptide comprising an amino acid sequence having amino acid sequence similarity of at least 60% (preferably at least 70%, more preferably at least 80%, still more preferably at least 90%, most preferably at least 95%) to the amino acid sequence of SEQ ID NO:1; (b) a polypeptide comprising an amino acid sequence derived from the amino acid sequence of SEQ ID NO:1 by deletion, substitution and/or addition of one or several amino acids (preferably not more than 20 amino acids, more preferably not more than 10 amino acids, still more preferably not more than 5 amino acids, yet more preferably 1, 2 or 3 amino acids) at one or several positions (preferably not more than five positions, more preferably not more than three positions, still more preferably not more than two positions, yet more preferably one position); and (c) a polypeptide comprising an amino acid sequence encoded by a nucleotide sequence capable of hybridizing under stringent conditions to a polynucleotide comprising a nucleotide sequence complementary to the nucleotide sequence of SEQ ID NO:2. Those analogues which are polypeptides having an activity to induce expansion of hematopoietic stem cells can be used as the HDGF peptide of this invention.

In a hHDGFa analogue used as the HDGF peptide of the present invention, the region at which the amino acid sequence of SEQ ID NO:1 is altered is not particularly limited, but the domain, motifs and the like, as mentioned above, which are believed to be responsible for the functions of hHDGFa, are preferably conserved. Such an analogue can be exemplified by analogues characterized in that, in the amino acid sequence of SEQ ID NO: 1, the amino acid sequences of positions 30 to 100 and 155 to 170 (more preferably the amino acid sequence of positions 30 to 208) are conserved with a sequence identity of preferably at least 80%, more preferably 90%, still more preferably 95%, most preferably 100%, and the amino acid sequence of any other region is altered. When the amino acid sequence of SEQ ID NO:1 is altered by substitution or addition of an amino acid(s), the amino acid(s) introduced by substitution or addition may be an amino acid(s) occurring in nature or an artificially synthesized amino acid(s), but is preferably an amino acid(s) occurring in nature. The "substitution" of an amino acid(s) is preferably a substitution by an amino acid(s) having similar properties to the amino acid (s) to be substituted, and examples of such a substitution include, but are not limited to, substitution of a hydrophobic amino acid by a different hydrophobic amino acid, substitution of a hydrophilic amino acid by a different hydrophilic amino acid, substitution of an acidic amino acid by a different acidic amino acid, or substitution of a basic amino acid by a different basic amino acid.

In Example 7 of the present specification, six truncated fragments of hHDGFa as shown in FIG. 13 (H1-208: the amino acid sequence of positions 1 to 208 of SEQ ID NO:1; H30-240: the amino acid sequence of positions 30 to 240 of SEQ ID NO:1; H30-208: the amino acid sequence of positions 30 to 208 of SEQ ID NO:1; H4-240: the amino acid sequence of positions 4 to 240 of SEQ ID NO:1; H1-230: the amino acid sequence of positions 1 to 230 of SEQ ID NO: 1; and H1-150: the amino acid sequence of positions 1 to 150 of SEQ ID NO:1) were analyzed for their expansion activity on hematopoietic stem cells, and the results demonstrated that all the fragments other than H1-150 maintain their expansion activity. This suggests that the region that plays an important role in the expansion activity of hHDGFa is the amino acid sequence of positions 30 to 208 of SEQ ID NO: 1. Therefore, the analogue of hHDGFa is preferably an analogue characterized in that the sequence homology of the aforementioned region is highly conserved, more preferably an analogue characterized in that at least 80% of the amino acid sequence of said region is conserved, still more preferably an analogue characterized in that at least 90% of the amino acid sequence of said region is conserved, yet more preferably an analogue characterized in that at least 95% of the amino acid sequence of said region is conserved, and most preferably an analogue characterized in that the amino acid sequence of said region is completely conserved. A preferred example of the HDGF analogue of this invention is a substance comprising the amino acid sequence of positions 30 to 208 of SEQ ID NO: 1, more preferably a peptide comprising the amino acid sequence of positions 1 to 208, positions 30 to 240, positions 30 to 208, positions 4 to 240, or positions 1 to 230, of SEQ ID NO: 1.

The source of production of the HDGF peptide used in the present invention is not particular limited -- for example, a culture supernatant or extract of HDGF peptide-expressing cells, or a HDGF peptide produced by a known genetic recombination technique, can be adopted. Specific examples of the HDGF peptide that can be used include, but are not limited to, peptides separated from a gene product expressed in a microorganism like *E. coli* or yeast, or insect or animal cells, having introduced therein a HDGF peptide-encoding gene, or from a gene product expressed in a cell-free system from wheat germ, etc. having introduced therein a HDGF peptide-encoding gene. In this invention, the HDGF peptide can be used regardless of the presence or absence of a sugar chain. The HDGF peptide with a sugar chain can be prepared by expressing it in animal cells like CHO cells. The HDGF peptide with no sugar chain can be prepared by expressing it in *E. coli.*

The HDGF peptide used in the present invention may be a naturally occurring peptide, or a peptide artificially designed based on the structure of native HDGF peptide a. The naturally occurring HDGF peptide is not limited to the human-derived peptides, hHDGFa, hHDGFb, and hHDGFc, and may be a HDGF peptide derived from any other animal, such as mouse HDGF (SEQ ID NO:5), bovine HDGF (SEQ ID NO:6), porcine HDGF (SEQ ID NO:7), chicken HDGF (SEQ ID NO:8), goat HDGF (SEQ ID NO:9), or sheep HDGF (SEQ ID NO:10). However, when used in human cells or human-derived cells, a peptide prepared by partially altering the structure of a human-derived HDGF peptide or a human HDGF peptide is preferably used.

The HDGF substance of the present invention includes a modified form of a HDGF peptide. In this invention, the "modified form of a HDGF peptide" refers to a substance that contains the amino acid sequence of the HDGF peptide with any other chemical structure being added thereto through chemical modification of part of the amino acids present in the HDGF peptide, and which has an activity to induce expansion of hematopoietic stem cells. Such a modified form can be exemplified by fusion proteins comprising a HDGF peptide, and conjugates of low-molecular or high-molecular compounds.

In the present invention, when the modified form of a HDGF peptide is a fusion protein comprising a HDGF peptide, it is only necessary that the HDGF peptide portion of the fusion protein have the desired sequence homology to the amino acid sequence of SEQ ID NO: 1, and an additional peptide portion need not be taken into account in terms of sequence homology. As long as the modified form of a HDGF peptide maintains the desired activity, any peptide can be selected as an additional peptide, but an additional peptide having *per se* no biological activity is generally selected. Typical examples of the additional peptide include, but are not limited to, IgG or a Fc fragment thereof, and albumin. The additional peptide may be added to the N-terminus, C-terminus, or both termini of the HDGF peptide in the fusion HDGF protein.

In the present invention, when the modified form of a HDGF peptide is modified by addition of a compound, any appropriate compound having the desired properties can be selected as the compound to be added, depending on the purpose of modification. For example, for the purpose of increasing peptide half-life in blood, a water-soluble polymer such as polyethylene glycol or sugar can be selected. When the HDGF peptide is degraded by a protease, degradation resistance can be imparted to the peptide by subjecting amino acids in a region cleaved by the protease to modification with a low-molecular compound. The amino acids modified in the HDGF peptide are not particularly limited as long as the desired functions are maintained, but amino acids other than those in the regions corresponding to the domain, motifs and the like, as mentioned above, which are believed to be responsible for the functions of HDGFa, are preferably modified. In such a modified form of a HDGF peptide, amino acids other than those in the regions of positions 30 to 100 and 155 to 170 in the amino acid sequence of SEQ ID NO:1 are preferably modified, and amino acids at the N-terminus, C-terminus, or both termini of said sequence are more preferably modified.

<In vitro use>

In one aspect of the present invention, there are provided, for example: a process for expanding hematopoietic stem cells, the process comprising contacting the expansion inducer of this invention in vitro with hematopoietic stem cells; a process for producing a biological material comprising expanded hematopoietic stem cells, the process comprising said expansion process; and a biological material comprising hematopoietic stem cells as produced by said production process. The aforementioned processes and biological material are very effective for conducting transplantation therapy or regenerative medicine using hematopoietic stem cells.

When used in in vitro expansion as mentioned above, the expansion inducer of the present invention can be provided in various forms depending on the site of study, culture or production, such as a reagent, additive, culture medium or the like comprising at least one HDGF substance as one constituent, or a substrate, such as culture vessel, having a HDGF substance immobilized on the surface. The concentration of the HDGF substance in a culture medium used in cell culture for hematopoietic stem cell expansion is not particularly limited as long as the concentration is enough to induce expansion of hematopoietic stem cells, and various concentrations of HDGF substance can be used. The lower limit of the HDGF substance concentration is preferably not less than about 0.1 ng/mL, more preferably not less than about 1 ng/mL, still more preferably not less than about 10 ng/mL. The upper limit of the HDGF substance concentration is preferably not more than about 1000 ng/mL, more preferably not more than about 500 ng/mL.

Cell culture aimed at such cell expansion can be conducted by applying common culture conditions for hematopoietic stem cells, except that the HDGF substance should be present in a culture medium.

The culture medium used in the processes of the present invention is not particularly limited, and various types of culture media, such as those commonly used in cell culture and those prepared for hematopoietic cell culture, can be used. Typical examples of culture media commonly used in cell culture include, but are not limited to, IMEM (Iscove's Modified Dulbecco's Medium), αMEM (alpha Modified Eagle's Minimum Essential Medium), DMEM (Dulbecco's Modified Eagle's Medium), Ham's F12 Medium (Ham's Nutrient Mixture F12), McCoy's 5A Medium, EMEM (Eagle's Minimum Essential Medium), and RPMI1640 Medium. Typical examples of serum-free culture media suitable for hematopoietic cell culture include, but are not limited to, StemPro34 (produced by Invitrogen), Stemline (produced by Sigma-Aldrich), Stemline II (produced by Sigma-Aldrich), X-VIVO 10 (produced by Lonza), X-VIVO 15 (produced by Lonza), X-VIVO 20 (produced by Lonza), HPGM (produced by Lonza), StemSpan H3000 (produced by STEMCELL Technologies), StemSpanSFEM (produced by STEMCELL Technologies), and QBSF-60 (produced by Quality Biological). Also, depending on the purpose, cell culture may be conducted under serum-free culture conditions, or may be conducted using culture conditions supplemented with a serum such as FBS or with a serum substitute such as Knockout Serum Replacement (produced by Gibco).

The period of cell culture aimed at hematopoietic stem cell expansion is not particularly limited. The lower limit of the culture period is not less than 1 hour, preferably not less than about 5 hours, more preferably not less than about 12 hours, still more preferably not less than about 24 hours, yet more preferably not less than about 48 hours. The upper limit of the culture period is not more than about 15 days, preferably not more than about 12 days, more preferably not more than about 10 days, still more preferably not more than about 7 days.

In the expansion induction process of the present invention, the HDGF substance can be used alone, or, from the viewpoint of increasing expansion induction efficiency, the HDGF substance may be used together with various components, such as cytokines, growth factors, chemicals, proteins, peptides, and antibodies, which serve to stimulate expansion and/or proliferation of hematopoietic stem cells. Examples of the cytokines used together with the HDGF substance in this invention include, but are not limited to, interleukin-1 (IL-1), IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35, IL-36, IL-37, IL-38, interferon-α (IFN-α), interferon-β (IFN-β), interferon-γ (IFN-γ), granulocyte colony-stimulating factors (G-CSF), granulocyte macrophage colony-stimulating factors (GM-CSF), macrophage colony-stimulating factors (M-CSF), erythropoietins (EPO), basic fibroblast growth factors (bFGF), acidic fibroblast growth factors (aFGF), insulin-like growth factors (IGF), epidermal growth factors (EGF), hepatocyte growth factors (HGF), transforming growth factors, platelet-derived growth factors (PDGF), cholinergic differentiation factors, leucocyte migration inhibitory factors (LIF), protease nexin I, protease nexin II, platelet-derived growth factors (PDGF), chemokine, Notch ligands (*e.g.*, Delta1), Wnt proteins, angiopoietin-like protein 2, 3, 5 or 7 (Angpt2, 3, 5, 7), insulin-like growth factors (IGF), insulin-like growth factor-binding proteins (IGFBP), pleiotrophin, stem cell factors (SCF), flk-2/flt-3 ligand (FL), thrombopoietin (TPO), IL-6/soluble IL-6 receptor complex, or IL-6/soluble IL-6 receptor fusion protein (Hyper IL-6). Also, any cytokines prepared by artificially altering the amino acid sequences of the above-listed cytokines by a genetic recombination technique may be used in combination. Among the aforementioned cytokines, stem cell factors (SCF), flk2/flt3 ligand (FL), thrombopoietin (TPO), granulocyte macrophage colony-stimulating factors (GM-CSF), interleukin-6 (IL-6), etc. are preferably used, and thrombopoietin (TPO) is more preferably used. The lower limit of the concentration of a cytokine used together in a culture medium during cell culture is preferably not less than about 0.1 ng/mL, more preferably not less than about 1 ng/mL, still more preferably not less than about 10 ng/mL. The upper limit of the cytokine concentration in a culture medium is preferably not more than about 1000 ng/mL, more preferably not more than about 500 ng/mL.

Examples of the chemicals that can be used together with the HDGF substance in the present invention include, but are not limited to, hydrocarbon antagonists, copper chelating agents, histone deacetylase inhibitors, DNA methylation inhibitors, retinoic acid receptor inhibitors, aldehyde dehydrogenase inhibitors, glycogen synthase kinase-3 inhibitors, prostaglandin $E_2$, and the thrombopoietin receptor agonist Eltronbopag. Also, other chemicals, proteins, peptides, antibodies, etc. which are capable of binding to the receptors of the aforementioned cytokines to display agonistic or antagonistic activity can be used. The foregoing chemicals or cytokines may be added to a culture medium, or may be immobilized on the surface of a substrate or carrier in a culture vessel.

The source of the hematopoietic stem cells used in the expansion process of the present invention is not particularly limited, and those stem cells of various sources can be adopted. Exemplary sources of hematopoietic stem cells include, but are not limited to, biological tissues such as bone marrow, umbilical cord blood, or peripheral blood, and hematopoietic stem cell-containing cell cultures produced in the process of inducing differentiation of pluripotent stem cells such as ES cells or iPS cells into blood cells. In particular, when intended for use in transplantation, hematopoietic stem cells are preferred to have been confirmed to have no impairment or abnormality before expansion. Such a tissue or sample may be applied as it is to the expansion process of this invention, or a cell population rich in hematopoietic stem cells may be selected and applied to the inventive expansion process. Such a cell population can be selected following a conventional procedure, for example, by fractionating CD34+ cells or CD34+CD38- cells with magnetic beads or a cell sorter using an antigen on the cell surface as an indicator.

After a hematopoietic stem cell source (*e.g.*, bone marrow cells, umbilical cord blood, peripheral blood) containing hematopoietic stem cells is cultured in the presence of a HDGF substance alone or both a HDGF substance and a cytokine, cells nonreactive with the HDGF substance or the cytokine, or dead cells may be found in large numbers, so that there may be no increase in the total number of cells as compared with that before culture. Even in such a case, the evidence of an increase in the number of CD34+ cells or CD34+CD38- cells suggests that hematopoietic stem cells have been expanded.

The cell population obtained after the induction of cell expansion in the present invention is a cell population comprising hematopoietic stem cells, hematopoietic progenitor cells, and/or mature blood cells which are produced by self-renewal of hematopoietic stem cells during culture. The hematopoietic progenitor cells and mature blood cells contained in said cell population can include those cells mixed in a hematopoietic stem cell source, or those cells produced by differentiation of hematopoietic stem cells.

The cell population obtained after culture using the expansion inducer of the present invention is a cell population comprising a mixture of expanded hematopoietic stem cells and differentiated hematopoietic stem cells. Expansion of hematopoietic stem cells as compared with before culture does not necessarily mean an increase in the number of these cells, but means an increase in the number of cells derived from these hematopoietic stem cells. When hematopoietic stem cells in a cell culture solution differentiate and convert into hematopoietic progenitor cells, mature blood cells, or other cells, the number of hematopoietic stem cells may remain unchanged or decrease. In such cases, hematopoietic stem cells will eventually disappear as long as those cells are not expanded. Thus, the continuous existence of hematopoietic stem cells *per se* suggests that hematopoietic stem cells have been expanded.

It was demonstrated in working examples that the hematopoietic stem cells expanded in the present invention maintain their bone marrow repopulating ability upon secondary transplantation, and that the expansion inducer of this invention is capable of inducing the expansion of hematopoietic stem cells into cells having suitable properties for hematopoietic stem cell transplantation. By using the expansion inducer of this invention, a stem cell source such as umbilical cord blood can be confirmed in advance as a stem cell source suitable for transplantation, through determination of whether the source contains hematopoietic stem cells appropriate for transplantation.

The hematopoietic stem cells expanded by the process of the present invention can be used as transplant cells. Since said hematopoietic stem cells include long-term hematopoietic stem cells having long-term bone marrow repopulating ability, said hematopoietic stem cells can be provided as a material for cell transplantation therapy. The transplant cells may be transplanted as they are, or may be transplanted after fractionation with magnetic beads or a cell sorter using an antigen on the cell surface as an indicator. The expanded hematopoietic stem cells may be transplanted into the same individual from which a hematopoietic stem cell source has been collected, or may be transplanted into a different individual. In other words, the hematopoietic stem cells expanded by the process of this invention can be used as a therapeutic graft in the same way as in conventional bone marrow transplantation, peripheral blood stem cell transplantation, and umbilical cord blood transplantation. For example, when a subject undergoing transplantation is human, a bone marrow or peripheral blood sample has been collected before operation from a solid cancer patient scheduled to undergo high-dose chemotherapy or a patient at risk for bone-marrow suppression due to radiation or other therapy, and hematopoietic stem cells present in the sample are expanded in vitro and transplanted back into the patient after operation, whereby hematopoietic disorders can be restored early.

Further, the present invention provides a process for identifying a biological material comprising hematopoietic stem cells, the process comprising the steps of: culturing at least part of a biological material containing cells in the presence of a HDGF substance; and confirming expansion of hematopoietic stem cells in the cultured cells. Hematopoietic stem cells are abundant in a cell population of CD34+ cells, particularly CD34+CD38- cells. However, these cell-surface markers are strictly mere indicators, and hematopoietic stem cells cannot be determined to actually function as such without confirmation of their pluripotency and self-renewal ability. In particular, self-renewal ability is essential for repopulation of the bone marrow, and is an important characteristic for use of hematopoietic stem cells in transplantation. For example, since pluripotent hematopoietic progenitor cells have pluripotency but are unable to self-renew, temporal restoration of hematopoietic potency by transplantation of said cells can be expected, but hematopoietic disorders will recur in the long run. Since the expansion inducer of this invention expands hematopoietic stem cells by inducing self-renewal of hematopoietic stem cells, the expansion inducer is also useful as a reagent for determination of the self-renewal ability of cells. In working examples given hereinbelow, cell samples observed to include CD34+ cells expanded by treatment with a HDGF substance were subjected to hematopoietic stem cell transplantation studies in NOG mice, and the bone marrow was found to be successfully repopulated even during secondary transplantation -- thus, the results demonstrated that the expanded cells were long-term hematopoietic stem cells.

Also, since hematopoietic stem cells are capable of differentiating into all types of blood cells, it is possible to induce hematopoietic stem cells to differentiate in vitro into different types of hematopoietic progenitor cells or different types of mature blood cells, and then to transplant such hematopoietic progenitor cells or mature blood cells into patients in need of HSC replacement. By doing such a transplantation, hematopoietic disorders in patients with deficiency in a particular type of mature blood cells can be ameliorated. In other words, the present invention further provides, for example: a process for producing hematopoietic progenitor cells or mature blood cells, the process comprising the step of inducing differentiation of hematopoietic stem cells expanded in vitro using the expansion inducer of this invention; and a biological material comprising the hematopoietic progenitor cells or mature blood cells produced by said production process. Hematopoietic progenitor cells and mature blood cells have no self-renewal ability and thus have been required to be collected in a necessary amount from the living body in conventional transplantation therapies. However, since this invention enables in vitro expansion of hematopoietic stem cells upstream of hematopoietic progenitor cells and mature blood cells, the desired hematopoietic progenitor cells or mature blood cells can be prepared in vitro in a necessary amount from a small amount of a hematopoietic stem cell source. To put it more specifically, hematopoietic progenitor cells can be obtained by culturing hematopoietic stem cells expanded using the inventive factor, in the presence of a factor such as SCF, FL or TPO and supporting cells to induce differentiation of the hematopoietic stem cells. Further, blood cells can be acquired by adding a factor required for maturation into different types of blood cells to a cell culture system of the induced hematopoietic progenitor cells. Examples of the factor to be added include G-CSF for granulocytes, EPO for erythrocytes, and TPO for platelets. Also, the effects of such factors can be enhanced by adding an enhancement factor such as IL-6 or IL-11.

<Subject undergoing therapy>

The subject to undergo a therapy using the expansion inducer for hematopoietic stem cells of the present invention (*i.e.,* transplantation therapy using prepared cells, or therapy involving administration of the expansion inducer as a medicament) can be exemplified by individuals suffering from diseases with symptoms caused by hematopoietic disorders or depressed or impaired functions of hematopoietic cells or blood cells, and individuals having such symptoms resulting from treatments of certain diseases. Such diseases include, but are not limited to, diseases associated with impaired hematopoietic function, diseases associated with depressed hematopoietic function, diseases associated with decreased hematopoietic cells, diseases associated with increased hematopoietic cells, diseases associated with decreased immune cells, diseases associated with increased immune cells, and diseases associated with autoimmunity.

Specific diseases include, but are not limited to, leukemias, myeloproliferative disorders, malignant lymphomas, plasmacytomas, solid tumors, aplastic anemia, pure red cell aplasia (PRCA), paroxysmal nocturnal hemoglobinuria (PNH), inborn error of metabolism, chronic active EBV infections, hemophagocytic syndrome (HPS), congenital hematopoietic disorders, Fanconi syndrome, chronic hepatic dysfunction, renal failure, severe infections, myelopathic thrombocytopenia, idiopathic thrombocytopenic purpura (ITP), STE, poisonous snakebite, hemolytic-uremic syndrome, hypersplenism, Barnard-Soulier syndrome, Glanzmann's thrombasthenia, uremia, chronic granulomatous disease, severe combined immunodeficiency, adenosine deaminase (ADA) deficiency, agammaglobulinemia, immunodeficiency syndromes such as Wiskott-Aldrich syndrome, Chediak-Higashi syndrome and acquired immunodeficiency syndrome (AIDS), C3 deficiency, congenital anemias such as thalassemia, hemolytic anemias caused by enzyme deficiencies, and sickle cell anaemia, lysosomal storage diseases such as Gaucher disease and mucopolysaccharidosis, and adrenoleukodystrophy.

Leukemias include, but are not limited to, acute myelocytic leukemia, acute lymphatic leukemia, chronic myelocytic leukemia, myelodysplastic syndrome, adult T-cell leukemia (ATL), and chronic lymphocytic leukemia. Malignant lymphomas include, but are not limited to, follicular lymphoma, aggressive lymphoma, and Hodgkin's lymphoma. Plasmacytomas include, but are not limited to, multiple myeloma.

Further, when patients receiving massive transfusion of stored blood or surgical massive transfusion, patients with massive bleeding, or patients with antiplatelet antibody are treated with hematopoietic stem cell transplantation or in vivo administration of the hematopoietic stem cell expansion inducer, a deficiency of blood cells can be replenished. In the case of a deficiency of a particular type of blood cells, it is also acceptable to transplant only the desired type of blood cells obtained by inducing differentiation from hematopoietic stem cells expanded according to the present invention. However, if there is a concern about a long-term deficiency of blood cells, transplantation of hematopoietic stem cells or in vivo administration of the hematopoietic stem cell expansion inducer is preferred.

Further, when a bone marrow or peripheral blood sample has been collected in advance from an individual not suffering from such a symptom or disease to store hematopoietic stem cells expanded by the expansion process of the present invention, the individual, if actually suffering from a disease, will be able to undergo transplantation of self-derived cells which are in no danger of being immunologically rejected.

The hematopoietic stem cells expanded according to the present invention can be used in regenerative medicine as gene therapy cells. Gene therapy is performed by transferring a therapeutic gene into the hematopoietic stem cells expanded by the process of this invention or expanding hematopoietic stem cells into which a therapeutic gene has been transferred, and then by transplanting the expanded hematopoietic stem cells into an individual. The therapeutic gene to be transferred in this process can be of any appropriate type selected depending on the disease to be treated, and is not particularly limitedexamples include hormone genes, cytokine genes, receptor genes, enzyme genes, polypeptide genes, and other genes. Gene therapy utilizing gene transfer into hematopoietic stem cells can be applied to the treatment of congenital genetic disorders. Such exemplary cases include, but are not limited to, transfer of the adenosine deaminase (ADA) gene for ADA deficiency (Blaese R.M., et al., Science. 1995; 270 (5235): 475-480), transfer of the γc chain gene for X-linked severe combined immunodeficiency (X-SCID) (Hacen-Bay-Abina S., et al., N Engl J Med. 2002; 346: 1185-1193), transfer of the glucocerebrosidase gene for chronic granulomatous disease (many Japanese CGD patients lack $gp91^{phox}$ and are treated by the transfer of this gene) or Gaucher's disease, and a therapy of hemophilia. Other effective genes are RNA genes that suppress disease gene expression, such as antisense RNA genes, siRNA genes, shRNA genes, Decoy RNA genes, and ribozyme genes.

<Medicinal use (in vivo use)>

In another aspect, the expansion inducer of the present invention is provided as a medicament comprising a HDGF substance as an active component. The HDGF substance, when administered in vivo, is capable of expanding hematopoietic stem cells present in the body of an individual administered with the substance; thus, if hematopoietic stem cells possessed by said individual have a normal function, expansion of the hematopoietic stem cells by the HDGF substance leads to restoration, alleviation or treatment of hematopoietic disorders or other symptoms.

The expansion inducer used as a medicament in the present invention is administered for the purpose of treating or alleviating a disease or symptom in the aforementioned individual to be treated. Also, when the expansion inducer of this invention is administered to a patient undergoing hematopoietic stem cell transplantation, the expansion inducer acts to promote engraftment of transplanted hematopoietic stem cells and to stimulate recovery of all types of blood cells including erythrocytes, platelets and leukocytes. Further, administration of the expansion inducer of this invention to a patient undergoing cytotoxic drug or radiation therapy can be expected to contribute to early recovery of hematopoietic function.

In one aspect, the present invention relates to a process for treating a disease caused by depressed or impaired hematopoietic function, the process comprising administering at least one HDGF substance to a subject. In another aspect, this invention relates to use of at least one HDGF substance in the production of a therapeutic agent for a diseased caused by depressed or impaired hematopoietic function. In still another aspect, this invention relates to use of at least one HDGF substance in the treatment of a disease caused by depressed or impaired hematopoietic function. Furthermore, this invention relates to a pharmaceutical composition for the treatment of a disease caused by depressed or impaired hematopoietic function, the pharmaceutical composition comprising at least one HDGF substance.

As the medicament of the present invention, the expansion inducer can be administered concurrently with any various cytokines or chemicals. The cytokine or chemical to be concurrently used can be selected as appropriate depending on various factors including the symptom or age of the patient. Such a cytokine or chemical may be incorporated in the same preparation with a HDGF substance, or may be administered as a different preparation from that of a HDGF substance, at the same time or a different time. Such a cytokine concurrently used with the medicament of this invention is, for example, preferably a cytokine having an activity to enhance or stabilize the expansion activity of a HDGF substance on hematopoietic stem cells, and can be exemplified by SCF, flt3L, TPO, EPO, IL-6 or the like.

The substance that can be used as an active component of the medicament of the present invention may be a pharmaceutically acceptable salt of a HDGF substance as mentioned above. In other words, in this invention, the aforementioned HDGF substance provided in the form of a salt having added thereto an acid such as an inorganic acid like hydrochloric acid, sulfuric acid or phosphoric acid, or an organic acid like formic acid, acetic acid, butyric acid, succinic acid or citric acid, can also be used as an active component. Alternatively, in this invention, the aforementioned HDGF substance provided in the form of a salt of a metal such as sodium, potassium, lithium or calcium, or a salt of an organic base, may also be used as an active component. Further, the pharmaceutical composition of this invention may be provided in a free form of a substance serving as an active component, or in the form of a pharmaceutically acceptable salt thereof.

The pharmaceutical composition of the present invention is prepared not only using a HDGF substance or a pharmaceutically acceptable salt thereof as an active component, but also using a carrier, an excipient, other additives, a diluent or the like commonly used in formulating pharmaceutical preparations. Examples of the carrier or excipient for use in pharmaceutical preparations include lactose, magnesium stearate, starch, talc, gelatin, agar, pectin, gum arabic, olive oil, sesame oil, cacao butter, ethylene glycol and other commonly used ones.

A solid composition for oral administration is used in the form of a tablet, pill, capsule, powder, granule or the like. In such a solid composition, at least one active component is mixed with at least one inactive diluent such as lactose, mannitol, glucose, hydroxypropyl cellulose, microcrystalline cellulose, starch, polyvinyl pyrrolidone, or magnesium aluminometasilicate. Such a composition may also contain other additives than an inactive diluent according to a common procedure -- for example, a lubricant such as magnesium stearate, a disintegrant such as calcium carboxymethylcellulose, and/or a solubilizer such as glutamic acid or asparatic acid. The tablet or pill, depending on the need, may be coated with a sugar coating made of saccharose, gelatin, hydroxypropylmethylcellulose phthalate or the like, or a film of a gastric- or enteric-soluble substance, or may be coated with two or more layers. Further, a capsule made of an absorbable substance such as gelatin is included as a solid composition.

A liquid composition for oral administration, which can be exemplified by a pharmaceutically acceptable emulsion, solution, suspension, syrup, elixir or the like, may comprise a commonly used inactive diluent such as purified water or ethanol. In addition to an inactive diluent, such a composition may further comprise an adjuvant such as moistening agent or suspending agent, a sweetener, a flavoring agent, an aromatic, an antiseptic, or the like.

An injection for parenteral administration can be exemplified by an aseptic aqueous or non-aqueous solution, suspension, or emulsion. The aqueous solution or suspension can be exemplified by an injection solvent or physiological saline for injection. The non-aqueous solution or suspension can be exemplified by a vegetable oil such as propylene glycol, polyethylene glycol, or olive oil, an alcohol such as ethanol, or Polysorbate 80™. Such a composition may further comprise an antiseptic, a moistening agent, an emulsifier, a dispersant, a stabilizer (*e.g.,* lactose), a solubilizer (*e.g.,* glutamic acid, asparatic acid), or the like. Such injections can be sterilized by, for example, filtration sterilization using a microfiltration membrane, heat sterilization such as high-pressure steam sterilization, or a common sterilization method such as addition of a disinfectant. The injection may be a solution preparation, or a free-dried injection for reconstitution into solution before use. As an excipient for freeze drying, a sugar alcohol or sugar such as mannitol or glucose can be used.

It is preferred that the pharmaceutical composition of the present invention be administered by an administration method commonly used in pharmaceutical applications, such as oral administration, or parenteral administration including transmucosal, intravenous, intramuscular or subcutaneous administration. When an active component is an antibody or protein, the pharmaceutical composition is commonly administered by a parenteral route of administration, such as injection (*e.g.*, subcutaneous, intravenous, intramuscular, or intraperitoneal injection), or percutaneous, transmucosal, transnasal or transpulmonary administration, or may be administered by an oral route.

When an active component is a peptide substance, the pharmaceutical composition can be orally administered as a preparation less susceptible to degradation in the gastrointestinal tract, such as a microcapsule having the active component peptide encapsulated in a ribosome. The pharmaceutical composition may also be administered by an administration method that causes absorption from other mucosae than that of the gastrointestinal tract, such as rectal, intranasal, sublingual, percutaneous, subcutaneous, intravenous, intraperitoneal, or intramuscular administration. In such cases, the pharmaceutical composition can be administered to an individual in the form of suppository, nasal spray, inhalation medicine, sublingual tablet, injection, intravenous fluid, suppository, percutaneous absorbent, transmucosal absorbent, or the like.

The dose of a substance usable as an active component of the pharmaceutical composition of the present invention varies with various factors including the type of the primary disease, the age, weight and severity of condition of the patient (subject), and administration route. For example, in the case of administration by injection to a human, the lower limit of the daily dose of a HDGF substance, in terms of protein amount, is about 0.0002 μg/kg, preferably about 0.002 μg/kg, more preferably about 0.02 μg/kg. And the upper limit of the daily dose of a HDGF substance adopted in this case is about 200 mg/kg, preferably about 20 mg/kg, more preferably about 2 mg/kg.

The pharmaceutical composition of the present invention may be concurrently used, or formulated in the same preparation, with any other active substances. Examples of other active substances concurrently used include colony-stimulating factors, cytokines, chemokines, interleukins, cytokine receptor agonists or antagonists, soluble receptors, receptor agonist or antagonist antibodies, or chemicals and peptides that act by the same mechanism as one or more foregoing active substances.

When applied to gene therapy, the therapeutic agent of the present invention can be used in the form of a product prepared by incorporating a nucleic acid(s) encoding the amino acid sequence of a HDGF peptide into a known medium suitable for gene therapy, such as a viral vector, preferably a lentiviral vector or an adeno-associated viral vector, more preferably an adenoviral vector, a chemically synthesized liposome, a viral envelope, or a complex of a viral envelope and a synthetic liposome, downstream of such a promoter sequence that functions in host cells, such as cytomegalovirus promoter (CMV promoter). As a gene encoding a HDGF peptide, there can be used a gene comprising a nucleotide sequence encoding an amino acid sequence of any of SEQ ID NOs:1-10. Specific examples of a gene therapy method that can be used include the method described in Experimental Medicine (1994, vol.12, p.303), or the methods of the publications cited therein.

## EXAMPLES

Hereunder, the present invention will be described in detail by way of specific experimental examples. However, the experimental examples given below are merely some embodiments of this invention and are not limitative of the invention. For the purpose of the present specification, unless otherwise stated, concentrations and the like are expressed on a mass basis, and numerical ranges include their endpoints. As referred to in the examples, "HDGF" refers to hHDGFa, unless otherwise specified.

<Example 1> Expansion study in hCD34+ cells

Analyses have been made on various biological samples to search for a factor capable of expanding hematopoietic stem cells, and as a result, a cell culture supernatant of COS-1 cells having introduced therein the RELA (v-rel avian reticuloendotheliosis viral oncogene homolog A) gene was observed to have potent expansion activity on hCD34+ cells. The results of chromatographic purification and mass spectrometry of the culture supernatant suggested the possibility that hHDGFa might serve as an active ingredient.

In this example, in order to confirm the expansion activity of a HDGF substance on hematopoietic stem cells, wheat germ-expressing recombinant hHDGFa protein used as a HDGF substance was determined for its expansion activity on hCD34+ cells.

Purified hHDGFa was prepared by reacting PreScission protease (produced by GE Healthcare) with a solution of GST-tagged wheat germ-expressing hHDGFa protein (produced by Abnova; containing the PreScission protease cleavage recognition sequence), passing the resulting cleavage reaction solution through a GSTrap column (produced by GE Healthcare), and separating a cleaved GST-tagged fragment, PreScission protease, and hHDGFa.

Human umbilical cord blood-derived CD34+ cells (produced by Lonza) were prepared in an αMEM medium (produced by Gibco) supplemented with 20% FCS (produced by Gibco) and 1% BSA (produced by Sigma) according to the protocol attached to the cell product. The prepared cells were suspended in the aforementioned medium and stored at 4°C for at least 72 hours. Then, the cell suspension was adjusted to a concentration of $1\times10^4$ cells/0.9 mL with the aforementioned medium supplemented with all of hSCF (final concentration of 100 ng/mL; produced by R&D), hFlt3-L (final concentration of 100 ng/mL; produced by R&D), hIL-6 (final concentration of 10 ng/mL; produced by R&D) and hGM-CSF (final concentration of 10 ng/mL; produced by R&D). 270 μL of the cell suspension was added to each well of a 48-well culture plate (produced by Falcon). Further, 30 μL each of the hHDGFa solutions adjusted to different concentrations (0 ng/mL, 5 ng/mL, 50 ng/mL, 500 ng/mL, 5000 ng/mL) with PBS(-) (produced by Sigma) containing 0.1% BSA (produced by Sigma) was added to each well of the plate, and the plate was cultured under the conditions of 37°C and 5% $CO_2$ for 7 days. After the culture, the cells were stained with trypan blue (produced by Gibco) to count living cells using a hemocytometer (FIG. 2). The results revealed that hHDGFa expanded CD34+ cells in a concentration-dependent manner over the range of 0.5 ng/mL to 500 ng/mL (final concentration).

<Example 2> CD34+ cell expansion and colony expansion assays

*E. coli*-expressing recombinant hHDGFa protein used as a HDGF substance was analyzed for its CD34+ cell expansion activity and its hematopoietic progenitor cell colony expansion activity.

(1) Preparation of *E. coli*-expressing recombinant hHDGFa protein

The cDNA of hHDGFa was inserted into a pTWIN1 vector (produced by New England Biolabs) to construct an expression vector (pTWIN1-HDGF-intein), and *E. coli* was transformed with the expression vector to acquire intein-hHDGFa-expressing *E. coli.* Next, the intein-hHDGFa-expressing *E. coli* was cultured and lysed, and intein-hHDGFa was captured from the cell lysate by a chitin resin. After washing, the intein tag was self-cleaved in the presence of DTT to prepare purified recombinant hHDGFa protein. The thus-prepared purified recombinant hHDGFa protein was used as a HDGF substance in Experiments 2 and 3.

(2) CD34+ cell expansion assay

Human umbilical cord blood-derived CD34+ cells (produced by Lonza) were prepared in the same way as in Experiment 1 and adjusted with a serum-free medium (StemPro-34; produced by Gibco) to make a cell suspension at a concentration of $1\times10^4$ cells/mL. 300 μL of the cell suspension was added to each well of a 48-well culture plate (produced by Falcon). Next, hHDGFa (final concentration of 25 ng/mL), hSCF, hFlt3L (FL), hTPO (for the above three, final concentration of 100 ng/mL; produced by R&D) and combinations thereof were each adjusted to 100-fold of their final concentrations to prepare different mixed solutions. 3 μL/mL each of the mixed solutions was added to each well of the plate, and the plate was cultured in an incubator under the conditions of 37°C and 5% $CO_2$. After 7 days of culture, the number of cells was counted using a hemocytometer, and part of each of the samples was subjected to colony assay. Since the amounts of the samples added were one hundredth of the total amount, the increased volumes were ignored. For each of the samples, the fold expansion of cultured CD34+ cells relative to initial CD34+ cells (Input) present at an initial cell concentration of $1\times10^4$ cells/mL was calculated. The results are shown in FIG. 3.

As a result of analysis of the effects of no cytokine addition (None), hSCF alone (S), hFL alone (F), hTPO alone (T), hSCF+hFL (SF), hSCF+hTPO (ST), hFL+hTPO (FT), hSCF+FL+hTPO (SFT), hHDGF alone (H), hHDGFa+hSCF (HS), hHDGFa+hFL (HF), hHDGFa+hTPO (HT), hHDGFa+hSCF+hFL (HSF), hHDGFa+hSCF+hTPO (HST), hHDGFa+hFL+hTPO (HFT), and hHDGFa+hSCF+hFL+hTPO (HSFT) on the expansion of hCD34+ cells, no CD34+ cells were detected after 7 days of culture in the absence of a cytokine, which suggested the necessity of a cytokine for maintenance of cell survival. Also, the expansion of CD34+ cells was observed after culture in the presence of each of different cytokines and combinations thereof. In particular, as for hHDGFa, the fold expansions for HF, HT, HS, HSF, HFT, HST and HSFT were higher than those for corresponding F, T, S, SF, FT, ST and SFT; thus, it is considered that hHDGFa promotes the expansion of CD34+ cells under the coexistence with at least any one of the cytokines SCF, FL and TPO.

(3) Colony expansion assay

Next, in order to investigate the colony-forming ability of cells cultured under different conditions, the cells collected from each well of the plate after 7 days of culture in the aforementioned cell expansion assay (uncultured initial cells were used as input) were seeded into a cocktail supplemented with 1.0% (final concentration) methylcellulose (produced by STEMCELL Technologies), hSCF (100 ng/mL; produced by R&D), hGM-CSF (100 ng/mL; produced by R&D), hTPO (50 ng/mL; produced by R&D), hIL3 (100 ng/mL; produced by R&D), hEPO (5 U/mL; produced by Kyowa Hakko Kirin), hG-CSF (100 ng/mL; produced by Kyowa Hakko Kirin), 30% FCS (produced by HyClone), 1% BSA (produced by Sigma), and IMDM (produced by Invitrogen). The cell contents were stirred well by vortexing and left to stand for 20 minutes, and 1 mL of the cell suspensions were transferred to 35-mm culture dishes (produced by Falcon). After the dishes were cultured in an incubator under the conditions of 5% $CO_2$ and 37°C for 17 days, the numbers of different colonies (CFU-Mix, BFU-E, CFU-GM) were counted in the dishes under a microscope. Also, the percentage of CFU-Mix relative to the total number of colonies (hereinafter referred to as "Mix percentage") was calculated for each group. The results are shown in Table 1.

The colony assay detects the following three types of hematopoietic progenitor cell colonies: the most immature CFU-Mix, and more differentiated BFU-E and CFU-GM. Since hematopoietic cells differentiate while undergoing cell division, the numbers of cells and cell colonies increase with the progress of differentiation. From the viewpoint of the activity of a factor capable of expanding hematopoietic stem cells, promoting cell differentiation is undesirable because this results in a decrease in the number of hematopoietic stem cells *per se,* and it is important to expand hematopoietic stem cells while maintaining the immature state of the stem cells as much as possible. Therefore, a higher Mix percentage is important.

As evident from the results shown in Table 1, the different types of colonies were observed to be formed from the uncultured initial cells, whereas no colony formation was observed in the cells (None) cultured in the absence of a cytokine. This demonstrated that a particular type of cytokine is required to maintain cells with colony-forming ability, such as hematopoietic stem cells and hematopoietic progenitor cells. Speaking of different cytokines, when the cells were cultured in the presence of SCF and/or hFlt3-L, the number of colonies formed was high but the Mix percentage was not so high -- in light of this, it is considered that differentiation of the cells might progress during culture. In contrast, there was a tendency that the cells cultured in the presence of HDGF and/or TPO showed a high Mix percentage. In the cells cultured in the presence of HDGF alone, the number of colonies formed was not so high but the Mix percentage was as high as 60%. In the cells cultured in the presence of HT, HST, HFT, or HSFT, the number of CFU-Mix was considerably increased and the Mix percentage was also increased, as compared with the Input cells and the corresponding cells cultured in the presence of T, ST, FT, or SFT. These results suggested that since CFU-Mix has no self-renewal ability, hHDGFa expanded hematopoietic stem cells and hematopoietic progenitor cells, which are more immature than CFU-Mix. Also, in this experiment, hTPO was observed to have a comparable level of activity to hHDGFa, and particularly in the HT group, which was cultured in the presence of both hHDGFa and hTPO, the Mix percentage was very high, *i.e.,* 77%. Therefore, the activities of hHDGFa and hTPO were analyzed in subsequence examples.

[Table 1]

Table 1. Numbers of different colonies and Mix percentage determined in the colony assay

| Culture conditions | CFU-GM | BFU-E | CFU-Mix | Mix percentage (%) |
|---|---|---|---|---|
| Input | 26.5 | 15 | 6.5 | 13.54 |
| None | 0.5 | 0 | 0 | 0 |
| S | 16.5 | 5.5 | 2.5 | 10.2 |
| F | 5.5 | 2.5 | 2.5 | 23.81 |

(continued)

| Culture conditions | CFU-GM | BFU-E | CFU-Mix | Mix percentage (%) |
|---|---|---|---|---|
| T | 2.5 | 4.5 | 6.5 | 48.15 |
| SF | 25 | 7.5 | 2 | 5.8 |
| ST | 24.5 | 10.5 | 11.5 | 24.73 |
| FT | 10.5 | 4.5 | 8 | 34.78 |
| SFT | 43.5 | 12 | 7.5 | 11.9 |
| H | 1 | 0 | 1.5 | 60 |
| HS | 17.5 | 6 | 6.5 | 21.67 |
| HF | 8 | 0.5 | 4.5 | 34.62 |
| HT | 2 | 3 | 17 | 77.27 |
| HSF | 26.5 | 11 | 10.5 | 21.88 |
| HST | 24 | 3 | 48.5 | 64.23 |
| HFT | 12 | 2.5 | 15 | 50.85 |
| HSFT | 46.5 | 7.5 | 57 | 51.35 |

<Example 3> Determination of the expansion rate of hematopoietic stem cells

This experiment analyzed the expansion activities of hHDGFa and hTPO on hematopoietic stem cells isolated from human umbilical cord blood.

(1) Preparation of human umbilical cord blood-derived CD34+ cells

Fresh human umbilical cord blood obtained from a donor with informed consent was diluted with a silica suspension (produced by IBL) to a concentration of 10% (V/V), and the dilution was gently stirred and incubated at 37°C for about one hour. After the dilution was layered onto a lymphoprep with a density of 1.077 (produced by Axis-Shield), density centrifugation was performed at 1600 rpm for 30 minutes to obtain a mononuclear cell fraction. Next, CD34+ cells were isolated using the Direct CD34 progenitor Cell Isolation Kit and MACS Separation columns (produced by Miltenyi).

(2) Expansion of hematopoietic stem cells and preparation of transplant cells

The above-isolated hCD34+ cells were adjusted with StemPro-34 serum-free medium (produced by Gibco) to a concentration of $1\times10^5$ cells/mL. Part of the adjusted cell suspension was diluted 2-fold with the same medium and used as a cell suspension for transplantation into the uncultured (UC) group. The remainder of the cell suspension, 800 $\mu$L, was added to a 24-well plate (produced by Falcon), and then *E. coli*-expressing hHDGFa (final concentration of 250 ng/mL) prepared in Example 2 as a HDGF substance, hTPO (final concentration of 100 ng/mL), or both of them were added. The cell suspensions were cultured in an incubator under the conditions of 5% $CO_2$ and 37°C for 3 days in the hHDGF alone-treated group (H), and for 5 days in the TPO alone-treated group (T) and the hTPO+hHDGFa-treated group (HT). After the culture, the numbers of cells were counted in the same way as in Experiment 1, and then the cells were all collected, washed with the same serum-free medium three times, and finally suspended in 1.6 mL of a serum-free medium. The cell suspensions were used as cells for transplantation into the different cultured groups (H, T and HT groups). Part of each of the prepared cell suspensions was subjected to antigen analysis by a flow cytometer (MACSQuant; produced by Miltenyi). The results of hCD34 and hCD38 antigen analysis of the prepared transplant cells are shown in FIG. 4. The total number of cells and the absolute number of hCD34+hCD38- cells are shown in Table 2. In the H and HT groups, the total number of cells was lower, but the number of hCD34+hCD38-cells was higher, than in the UC group; thus, it was considered that hematopoietic stem cells were expanded.

[Table 2]

Table 2. Cell count analysis of the transplant cells cultured under different conditions and transplanted into one mouse

| Culture conditions | UC group | H group | T group | HT group |
|---|---|---|---|---|
| Total number of cells ($x10^4$) | 1 | 0.3 | 0.4 | 0.95 |
| hCD34+hCD38- cells ($x10^3$) | 0.89 | 1.13 | 0.11 | 1.76 |

(3) Transplantation into immunodeficient mice (NOG mice)

Immunodeficient mice (NOG mice; produced by the Central Institute for Experimental Animals) were irradiated with 1.2 Gy $\gamma$ rays twice at 4-hour intervals using the Gammacell 40 Exactor System (produced by Best Theratronics) to destroy their bone marrow. After the second irradiation, 200 $\mu$L of the transplant cell suspension prepared above was administered via tail vein to mice of different groups (UC, H, T and HT groups; 5 mice per group). The number of cells administered was $1\times10^4$ cells/mouse as calculated in terms of the number of uncultured hCD34+ cells.

(4) Analysis of human-derived cells in transplanted mice

At 23 weeks after the transplantation, the mice were anesthetized to collect blood samples and euthanized to collect bone marrow cells from left and right femurs. To detect different types of human blood cells in the collected bone marrow cells, the bone marrow cells were stained with hCD45 antibody (Pacific Blue; produced by BioLegend), mCD45 antibody (FITC; produced by BioLegend), hCD3 antibody (APC; produced by BioLegend), hCD19 antibody (PE-Cy5; produced by BioLegend), hCD33 antibody (PE; produced by BioLegend), hCD235a antibody (PE; produced by BioLegend), hCD34 antibody (APC; produced by BioLegend), and hCD38 antibody (PE; produced by BioLegend). To detect erythrocytes in the bone marrow, the bone marrow cells were stained with TER119 antibody (APC; produced by BioLegend) and hCD235a antibody (FITC; produced by BioLegend). After the staining, the bone marrow cells were hemolyzed in Pharm Lyse (produced by BD Bioscience), washed with PBS(-) (produced by Sigma) containing 0.5% BSA (produced by Sigma), and then subjected to antigen analysis using the aforementioned flow cytometer. The peripheral blood samples were stained with hCD41 antibody (APC; produced by BioLegend) to detect human platelets, hemolyzed and fixed using Lysing Solution (produced by BD Bioscience), and then analyzed by the flow cytometer. The results of FACS analysis of hCD34+hCD38- cells are shown in FIGs. 5, 6 and Table 3.

(5) Determination of the bone marrow repopulating ability and expansion of hematopoietic stem cells

In the process of determination of bone marrow repopulating ability, when different types of blood cells derived from the transplanted cells were detected and also hCD34+hCD38- cells, which are a fraction of hematopoietic stem cells, were observed, it was determined that cells with bone marrow repopulating ability were present in the transplanted cells. In the process of determination of the expansion of hematopoietic stem cells, when in mouse bone marrow samples at 23 or more weeks after the transplantation, the absolute number of hCD34+hCD38- cells in any of the different cultured groups was increased as compared with that in the UC group, it was determined that expansion activity was observed in that cultured group. The expansion rate of hematopoietic stem cells was calculated by dividing the absolute number of hCD34+hCD38- cells in each cultured group by the absolute number of hCD34+hCD38- cells in the UC group.

As a result of the analysis of bone marrow repopulating ability, at 23 weeks after the transplantation, human-derived T-cells (hCD3+ cells), B-cells (hCD19+ cells), granulocytes/monocytes (hCD33+ cells), erythroblasts (hCD235a+CD45- cells), erythrocytes (hCD235a+ cells), and platelets (hCD41+ cells) were detected in all the mice of the UC and HT groups and in 2 of 4 mice of the H group (one mouse died during the feeding period)pluripotency was observed in the three groups. On the other hand, no pluripotency was observed in any of the mice of the T group.

As calculated based on the results shown in Table 3 and FIG. 6, the absolute number of hCD34+hCD38- cells in bone marrow cells was determined to be $0.51\times10^3$ cells in the UC group, $1.73\times10^3$ cells in the H group, 0 cell in the T group, or $2.14\times10^3$ cells in the HT group. The expansion rate was determined to be about 3.4-fold in the H group, or about 4.2-fold in the HT group, whereas no expansion was observed in the T group. This revealed that the number of hCD34+hCD38- hematopoietic stem cells was decreased after culture in the presence of hTPO alone, whereas the number of hCD34+hCD38- hematopoietic stem cells was increased after culture in the presence of hHDGFa -- *i.e.,* in the presence of hHDGFa alone or hHDGFa+hTPO.

The results demonstrated that hCD34+hCD38- cells cultured in the presence of HDGF have bone marrow repopulating ability, and that hematopoietic stem cells capable of withstanding transplantation were expanded by such a culture.

[Table 3]

Table 3. Number of hCD34+hCD38- cells in mouse bone marrow at 23 weeks after transplantation (x$10^3$)

| | UC | H | T | HT |
|---|---|---|---|---|
| 1 | 0 | 3.56 | 0 | 1.0 |
| 2 | 0 | 3.35 | 0 | 1.26 |
| 3 | 0 | 0 | 0 | 1.30 |
| 4 | 0 | 0 | 0 | 3.74 |
| 5 | 2.56 | | 0 | 3.39 |
| a.v. | 0.51 | 1.73 | 0 | 2.14 |

<Example 4> Analysis of long term hematopoietic stem cell expansion

There are two types of hematopoietic stem cells: long-term hematopoietic stem cells and short-term hematopoietic stem cells. The most clinically important hematopoietic stem cells are long-term hematopoietic stem cells. Thus, hematopoietic stem cells expanded in the presence of both hHDGF and hTPO (hereinafter referred to as "HT-expanded human hematopoietic stem cells") were secondarily transplanted into mice to analyze the expansion of long-term hematopoietic stem cells in the expanded cells.

(1) Primary transplantation study

Primary transplantation was performed in the following two groups -- the uncultured group (UC group) and the group cultured with hHDGFa+hTPO for 5 days (HT group) -- by following the same procedure as in Example 3. The results of antigen analysis of the prepared transplant cells are shown in FIG. 7 and Table 4. In the HT group transplant cells, the total number of cells increased 1.25 times and the number of hCD34+hCD38- cells increased about 3 times, as compared with the UC group transplant cells.

[Table 4]

Table 4. Numbers of cells in primary transplant cells transplanted into one mouse

| | UC | HT |
|---|---|---|
| Total number of cells (x$10^4$) | 1 | 1.25 |
| Number of hCD34+hCD38- cells (x$10^3$) | 1.6 | 5.0 |

The thus-prepared UC group transplant cells and HT group transplant cells were transplanted into irradiated NOG mice (n = 8 per group). At 24 weeks after the transplantation, bone marrow cell samples were collected from the mouse femur of the two groups, and a half of the samples was used as secondary transplant cells and the other half was used for analysis. The bone marrow cell samples collected from the two groups were labeled with antibodies against hCD45, hCD33, hCD19, hCD3, hCD235a, mCD45 or mCD235a. Also, peripheral blood samples were collected from the mice of the two groups and labeled with antibodies against hCD41 or mCD41.

The cells thus labeled with different antibodies were analyzed by a flow cytometer by following the same procedure as in (4) of Experiment 3. The results are shown in FIG. 8 and Table 5.

[Table 5]

Table 5. Pluripotency analysis in mice after primary transplantation

| Primary transplantation | % hCD45+ | | % hCD33+ | | % hCD19+ | | % hCD3+ | | % human erythrocytes | | % human platelets | |
|---|---|---|---|---|---|---|---|---|---|---|---|---|
| | UC bone marrow | HT bone marrow | UC bone marrow | HT bone marrow | UC bone marrow | HT bone marrow | UC bone marrow | HT bone marrow | UC bone marrow | HT bone marrow | UC peripheral blood | HT peripheral blood |
| 1 | 61.84 | 70.33 | 20.77 | 21.19 | 35.31 | 16.4 | 2.21 | 32.34 | 2.64 | 24.68 | 2.87 | 2.13 |
| 2 | 45.49 | 71.61 | 25.68 | 14.58 | 6.12 | 53.09 | 12.11 | 1.28 | 2.01 | 0.92 | 1.34 | 0.77 |
| 3 | 74.81 | 90.34 | 13.53 | 17.63 | 53.14 | 67.62 | 3.43 | 1.25 | 14 | 9.09 | 5.73 | 0.84 |
| 4 | 80.12 | 84.57 | 44.91 | 9.91 | 32.14 | 71.76 | 1.20 | 0.84 | 4.96 | 1.66 | 1.47 | 0.95 |
| 5 | 73.79 | 83.5 | 20.26 | 7.51 | 51.14 | 71.95 | 0.71 | 1.12 | 4.06 | 0.28 | 0.39 | 0.37 |
| 6 | | 92.94 | | 34 | | 55.65 | | 1.34 | | 21.02 | | 1.22 |
| 7 | | 93.47 | | 32.04 | | 58.34 | | 0.73 | | 15.03 | | 1.15 |
| 8 | | 91.25 | | 24.89 | | 61.84 | | 2.23 | | 14.14 | | 1.11 |
| a.v. | 67.21 | 84.75 | 25.03 | 20.22 | 35.57 | 57.08 | 3.93 | 5.14 | 5.53 | 10.85 | 2.36 | 1.07 |

In Table 5, the percentages of hCD45+ cells (% hCD45+), hCD33+ cells (% hCD33+), hCD19+ cells (% hCD19+), and hCD3+ cells (% hCD3+), relative to all leukocytes (hCD45+ cells plus mCD45+ cells), in mouse bone marrow cells, were calculated by the following equations.

- Percentage of hCD45+ cells (% hCD45+)

= percentage of hCD45+ cells / (percentage of hCD45+ cells + percentage of mCD45+ cells) × 100

- Percentage of hCD33+ cells (% hCD33+)

= percentage of hCD33+ cells / (percentage of hCD45+ cells + percentage of mCD45+ cells) × 100

- Percentage of hCD19+ cells (% hCD19+)

= percentage of hCD19+ cells / (percentage of hCD45+ cells + percentage of mCD45+ cells) × 100

- Percentage of hCD3+ cells (% hCD3+)

= percentage of hCD3+ cells / (percentage of hCD45+ cells + percentage of mCD45+ cells) × 100

Also, % human erythrocytes and % human platelets were calculated by the following equations. It should be noted that calculation of different percentages was done using %T (Total).

- Percentage of human erythrocytes relative to all erythrocytes in mouse bone marrow cells (% human erythrocytes)

= percentage of human erythrocytes / (percentage of human erythrocytes + percentage of mouse erythrocytes) × 100

- Percentage of human platelets relative to all platelets in mouse peripheral blood (% human platelets)

= percentage of human platelets / (percentage of human platelets + percentage of mouse platelets) × 100

In the bone marrow cell samples from all the mice of the HT and UC groups, human T-cells (hCD3+ cells), B-cells (hCD19+ cells), granulocytes/monocytes (hCD33+ cells), erythroblasts (hCD235a+hCD45- cells), and erythrocytes (hCD235a+ cells) were detected. Also, human platelets (hCD41+) were detected in the analysis of the peripheral blood samples. In other words, pluripotency was observed in all the mice of both UC and HT groups after primary transplantation.

As a result of analysis of hCD34+hCD38- cells in the bone marrow cell samples of the two groups (the results are shown in FIG. 9 and Table 6), hCD34+hCD38- cells were found in the bone marrow cell samples from all the mice of both HT and UC groups, but the averaged expansion rate of hCD34+hCD38- cells in the HT group was about 6-fold of that of the UC group. In other words, it was demonstrated that human hematopoietic stem cells treated in the presence of both HDGF and TPO have stayed alive in an immature state in mouse bone marrow for 24 weeks, and at the same time have been expanded.

[Table 6]

Table 6. Absolute number of hCD34+hCD38- cells in mouse bone marrow after primary transplantation (x$10^3$)

| No. | UC group | HT group |
|---|---|---|
| 1 | 4.23 | 4.4 |
| 2 | 3.32 | 39.68 |
| 3 | 1.0 | 92.16 |
| 4 | 19.19 | 62.22 |
| 5 | 12.6 | 60.28 |
| 6 | Dead | 37.7 |
| 7 | Dead | 68.85 |
| 8 | Dead | 33.99 |
| a.v. | 8.07 | 49.91 |

(2) Secondary transplantation study

To conduct secondary transplantation, the other half of the aforementioned cells obtained from mouse bone marrow samples at 24 weeks after primary transplantation was administered via tail vein to NOG mice irradiated with γ rays by following the same procedure as upon primary transplantation. The groups secondarily transplanted with bone marrow cells from the UC and HT groups were described as "UC-2 group" and "HT-2 group", respectively. The peripheral blood and bone marrow cell samples collected from the NOG mice at 20 or more weeks after the secondary transplantation were analyzed by the same procedure as described above. Determination of the bone marrow repopulating ability and expansion rate of hematopoietic stem cells was conducted by the same procedure as in Example 3. The results of the pluripotency analysis are shown in FIG. 10 and Table 7, and the results of the analysis of hematopoietic stem cell expansion were shown in FIG. 11 and Table 8. Additionally, the different percentages shown in Table 7 were calculated by the same procedure as in (1) above.

[Table 7]

Table 7. Pluripotency analysis in mice after secondary transplantation

| Secondary transplantation | % hCD45+ | | % hCD33+ | | % hCD19+ | | % hCD3+ | | % human erythrocytes | | % human platelets | |
|---|---|---|---|---|---|---|---|---|---|---|---|---|
| | UC-2 bone marrow | HT-2 bone marrow | UC-2 bone marrow | HT-2 bone marrow | UC-2 bone marrow | HT-2 bone marrow | UC-2 bone marrow | HT-2 bone marrow | UC-2 bone marrow | HT-2 bone marrow | UC-2 peripheral blood | HT-2 peripheral blood |
| 1 | 60.83 | 84.80 | 26.32 | 29.02 | 21.57 | 51.84 | 13.66 | 0.61 | 21 | 7.81 | 0.23 | 1.53 |
| 2 | 58.27 | 40.67 | 21.90 | 13.20 | 34.35 | 26.33 | 0.03 | 0.14 | 1.8 | 1.29 | 0.55 | 0.06 |
| 3 | 11.47 | 89.41 | 3.42 | 21.11 | 0.02 | 64.12 | 8.95 | 0.51 | 7.48 | 10.68 | 0.38 | 0.34 |
| 4 | 39.01 | 60.25 | 22.17 | 18.26 | 16.31 | 38.67 | 0.08 | 1.42 | 3.07 | 4.22 | 0 | 0.1 |
| 5 | 3.784 | 83.56 | 1.93 | 13.53 | 1.81 | 66.75 | 0 | 0.01 | 2.68 | 1.83 | 0 | 0.26 |
| 6 | | 72.75 | | 40.22 | | 28.94 | | 1.30 | | 7.29 | | 0.67 |
| 7 | | 45.91 | | 45.66 | | 8.26 | | 0.04 | | 10.6 | | 0.71 |
| 8 | | 82.73 | | 37.46 | | 55.88 | | 0.68 | | 3.63 | | 0.77 |
| a.v. | 34.22 | 70.01 | 15.15 | 27.31 | 14.81 | 42.86 | 4.54 | 0.59 | 7.21 | 5.92 | 0.23 | 0.56 |

As a result of the pluripotency analysis, human hematopoietic cells were found in all the mice of the UC-2 group, but some of the mice were deficient in particular types of cells and displayed a biased hematopoiesis -- so, pluripotency was not observed in all the mice of this group. On the other hand, all different types of human blood cells were detected, and thus pluripotency was observed, in all the mice of the HT-2 group.

As a result of the analysis of hCD34+hCD38- cells, which are a fraction of hematopoietic stem cells (FIG. 11, Table 8), hCD34+hCD38- cells were detected in only 3 of 5 mice of the UC-2 group but in all the mice of the HT-2 group. Also, when the absolute numbers of hCD34+hCD38- cells in the UC-2 and HT-2 groups were compared by reference to Table 8, the averages of said cell counts in these groups were determined to be $0.99\times10^3$ cells and $30.27\times10^3$ cells, respectively, and found to be dramatically different from each other -- they differed in expansion rate by about 30-fold. The results suggest that even after secondary transplantation, long-term human hematopoietic stem cells capable of maintaining bone marrow repopulating ability were successfully expanded by culture in the presence of both HDGF and TPO.

In this experiment, a xenotransplantation study was conducted for almost one year from primary transplantation to secondary transplantation, and as a result it was found that HT-expanded human hematopoietic stem cells are able to repopulate the bone marrow not only after primary transplantation but also after secondary transplantation. This suggests that the expanded cells are long-term hematopoietic stem cells with long-term hematopoietic ability and are clinically applicable hematopoietic stem cells. At present, there has been no case where long-term hematopoietic stem cells were successfully expanded in such large amounts.

[Table 8]

Table 8. Absolute number of hCD34+hCD38- cells in mouse bone marrow after secondary transplantation ($x10^3$)

| No. | UC group | HT group |
|---|---|---|
| 1 | 1.04 | 62.08 |
| 2 | 2.8 | 14.22 |
| 3 | 0 | 13.44 |
| 4 | 1.09 | 12.11 |
| 5 | 0 | 29.12 |
| 6 | Dead | 25.49 |
| 7 | Dead | 39.46 |
| 8 | Dead | 46.26 |
| a.v. | 0.99 | 30.27 |

<Example 5> Effects of recombinant hHDGFa protein administration to human hematopoietic stem cell-transplanted mice

This example analyzed the effects of a HDGF substance on hematopoietic stem cells in the body of animals administered with this substance. As a HDGF substance, animal cell-expressing recombinant hHDGFa protein was used.

(1) Preparation of animal cell-expressing recombinant hHDGFa protein

The cDNA of hHDGFa was inserted into a pEXPR-IBA17 vector (produced by IBA GmbH) to construct a hHDGFa expression vector. The expression vector was transfected into Expi293F cells (produced by Life Technologies), which were then cultured under the conditions described in the protocol attached to the cell product, to thereby obtain a Strep-tag II-HDGF-expressing culture supernatant. After the resulting culture supernatant was adjusted to pH 8.0 with 1 mol/L Tris-HCl, biotin in the culture supernatant was deactivated with egg white avidin, captured with a StrepTrap HP column (produced by GE Healthcare) and washed, and then Strep-tagII-hHDGFa protein was eluted with a buffer containing 2.5 mM D-desthibiotin.

(2) Administration of hHDGFa to human hematopoietic stem cell-transplanted mice and analysis of this administration

CD34+ cells isolated from human umbilical cord blood were transplanted into $\gamma$ ray-irradiated NOG mice by administering a cell suspension to the mice via tail vein at a dose of $1 \times 10^4$ cells per mouse. The mice were measured for their weight and divided into four groups (n = 5 per group) such that the averaged weights of these groups were almost comparable. For 5 consecutive days from the day following transplantation, the mice were subcutaneously administered with hHDGFa at a dose of 10 μg, 1 μg or 0.1 μg per mouse (the concentrations were adjusted with 0.1% BSA-containing PBS; the control group was administered with a vehicle only). At 4 weeks after the transplantation, blood samples were collected from the tail vein of the mice under anesthesia, and stained with hCD45 antibody (Pacific Blue; produced by BioLegend), mCD45 antibody (FITC; produced by BioLegend), hCD3 antibody (APC; produced by BioLegend), hCD19 antibody (PE-Cy5; produced by BioLegend), hCD33 antibody (PE; produced by BioLegend), and hCD41 antibody (APC; produced by BioLegend). After the staining, for detection of human leukocytes, peripheral blood samples were hemolyzed in Pharm Lyse (produced by BD Bioscience) and washed once with a PBS(-) (produced by Sigma) solution supplemented with 0.5% BSA (produced by Sigma) and 2 mM EDTA (produced by Gibco). The washed samples were passed through a 0.45-μm mesh (produced by Falcon) and subjected to antigen analysis using a flow cytometer (MACSQuant; produced by Miltenyi). The percentage of hCD45+ cells in all leukocytes of the mouse peripheral blood (percent chimeras) was calculated according to the following formula: percentage of hCD45 / (percentage of hCD45 + percentage of mCD45) × 100. For detection of platelets, peripheral blood samples were fixed for about 1 hour by adding Lysing Solution (produced by BD Bioscience), washed once with FACS Buffer, passed through a 0.45-μm mesh (produced by Falcon), and then analyzed using a flow cytometer (MACSQuant; produced by Miltenyi). The results are shown in FIG. 12 and Table 9.

As a result of the determination of the percentage of hCD45+ cells (leukocytes) in the mouse peripheral blood samples of the different groups (percent chimeras) and the flow cytometric analysis of different types of detected human blood cells at 4 weeks after the transplantation, it was found that the percent chimeras of the control group was 1.77% whereas those of the HDGF-treated groups were 3.14% for the 0.1 μg group (1.77-fold of control group), 2.75% for the 1 μg group (1.55-fold of control group), and 3.56% for the 10 μg group (2.01-fold of control group). Detection of human platelets was observed in 2 of 5 mice of the control group, all 5 mice of the 0.1 μg group, 4 of 5 mice of the 1 μg group, and all 5 mice of the 10 μg group. All the mice treated with a HDGF substance in this study were found to show early recovery of human leukocytes. Also, all the treated groups showed better outcomes than the control group with regard to early recovery of platelets. This suggests that administration of a HDGF substance contributes to recovery of hematopoiesis during the initial stage of transplantation.

[Table 9]

Table 9 Percentages of hCD45+ cell chimeras in peripheral blood samples of different groups

| Individual | Control group | 0.1 μg group | 1 μg group | 10 μg group |
|---|---|---|---|---|
| 1 | 1.33 | 2.47 | 3.24 | 1.87 |
| 2 | 1.78 | 3.32 | 3.63 | 2.79 |
| 3 | 1.02 | 4.5 | 1.84 | 2.83 |
| 4 | 2.16 | 2.48 | 2.78 | 4.68 |
| 5 | 2.57 | 2.93 | 2.28 | 5.65 |
| a.v. | 1.77 | 3.14 | 2.75 | 3.56 |
| Ratio* | 1 | 1.77 | 1.55 | 2.01 |

*) This refers to a ratio of the averaged percentage of each group relative to that of the control group, which is taken as 1.

<Example 6> Analysis of the period of culture in the presence of HDGF+TPO

This example analyzed what effects different culture periods in the presence of HDGF+TPO have on the bone marrow repopulation activity demonstrated in Example 3 to be present in the CD34+ cells expanded in the presence of HDGF+TPO.

(1) Experimental method

Transplant cells were prepared by the same procedure as in (1) and (2) of Example 3. After HDGF and TPO were added with 100 μg/mL being used as a final HDGF concentration, the following three groups were prepared by performing cell culture for different days: 3-day cultured group (3d group), 5-day cultured group (5d group), and 7-day cultured group (7d group). The UC group adopted in Example 3 was used as a 0d group.

The different groups of transplant cells (0d, 3d, 5d and 7d groups) prepared by the aforementioned procedure were transplanted into NOG mice by following the same procedure as in (3) of Example 3.

At 12 weeks after the transplantation, the percentages of human cells (derived from the transplanted hCD34+ cells) in each of the peripheral blood cells and bone marrow cells of the mice undergoing transplantation were analyzed by following the same procedure as in (4) of Example 3.

(2) Results

As a result of the analysis of the bone marrow cells collected from the mice at 12 weeks after the transplantation, hCD45+ cells were detected in 4 of 5 mice of the 0d group but in all the mice of the other groups. The averaged percentages of hCD45+ cell chimeras in the bone marrow samples of the 0d, 3d, 5d and 7d groups were 26.15%, 70.34%, 76.93% and 40.82%, respectively - the lowest value was recorded in the 0d group, and the highest was recorded in the 5d group.

As a result of pluripotency analysis, hCD33+ cells (myelocytes) and hCD19+ cells (B-cells) were detected in 4 of 5 mice of the 0d group but in all the mice of the other groups. Also, hCD3+ cells (T-cells) were detected in 1 of 5 mice (0d group), 2 of 5 mice (3d group), 2 of 5 mice (5d group), and 0 of 5 mice (7d group); and hCD235a+ cells (erythroblasts) were detected in 3 of 5 mice (0d group), 5 of 5 mice (3d group), 5 of 5 mice (5d group), and 2 of 5 mice (7d group). Human erythrocytes were detected in 2 of 5 mice (0d group), 5 of 5 mice (3d group), 5 of 5 mice (5d group), and 5 of 5 mice (7d group).

As a result of the analysis of the peripheral blood cells collected from the mice at 12 weeks after the transplantation, human platelets were detected in 2 of 5 mice (0d group), 5 of 5 mice (3d group), 5 of 5 mice (5d group), and 5 of 5 mice (7d group).

The absolute numbers of hCD34+hCD38- cells, which are a fraction of hematopoietic stem cells, were analyzed and determined to be $7.10\times10^3$ cells (0d group), $14.96\times10^3$ cells (3d group, fold expansion: 2.1), $24.98\times10^3$ cells (5d group, fold expansion: 3.52), and $22.58\times10^3$ cells (7d group, fold expansion: 3.19).

The results of the present analyses revealed that the frequency of detection of T-cells was low in all of the groups, but the reason for this might be that the period of 12 weeks after transplantation was too short as it takes a long time to induce differentiation of T-cells. Also, erythrocytes and platelets were both detected in only 2 of 5 mice of the 0d group, but the reason for this might be because of delay in recovery of hematopoiesis due to low number of engrafted hematopoietic stem cells. The number of hCD34+CD38- cells in vitro increased more in the 3d, 5d and 7d groups than in the 0d group, and the most increase was found in the 5d group. There seemed to be no difference between the 0d and 3d groups in terms of the number of hCD34+CD38- cells, but the total number of cells decreased on day 3 of culture. This suggests that hCD34+ cells on day 0 included those cells which were dead or nonreactive with HDGF+TPO, and the results for the 0d and 3d groups as mentioned above might also be explained by the fact that hCD34+CD38- cells included those nonreactive or dead cells (the number of actually reactive hCD34+hCD38- cells is presumed to be at the level indicated by a red dotted line). From the results given above, it was demonstrated that there is a correlation between increased number of hCD34+CD38- cells in vitro and the absolute number of hCD34+CD38- cells in the bone marrow in vivo, and that the appropriate culture period for expansion of hematopoietic stem cells in the presence of HT is 5 days.

<Example 7> Analysis of in vitro activities of different truncated fragments of hHDGFa

(1) Preparation of different truncated fragments of hHDGFa

In order to obtain the information on the essential sequence for the expansion activity of hHDGF, six different truncated fragments of hHDGFa (H1-208, H30-240, H30-208, H4-240, H1-230 and H1-150; their structures are shown in FIG. 13) were prepared and used to perform CD34+CD38- cell expansion assay.

(2) Experimental method

Human umbilical blood-derived CD34+ cells were adjusted with a serum-free medium (StemPro-34; produced by Gibco) to yield a cell concentration of $1\times10^5$ cells/mL and a final concentration of hTPO (produced by R&D) of 100 ng/mL. After 300 μL of the adjusted cell suspension was added to a 48-well plate (produced by Falcon), 3 μL each of solutions of full-length hHDGFa or any of different truncated hHDGF fragments, which were adjusted to 50 μg/mL, was added to each well of the plate, and the plate was cultured in an incubator under the conditions of 5% $CO_2$ and 37°C for 5 days. After the culture, part of the samples was collected, stained with trypan blue, and counted for the number of living cells with a hemocytometer (C-Chip; produced Digital Bio). Then, all cells were collected, stained with an anti-hCD34 antibody (APC; produced by BioLegend) and an anti-hCD38 antibody (FITC; produced by BioLegend), and subjected to antigen analysis using a flow cytometer (MACSQuant; produced by Miltenyi). Dead cells were removed using PI (propidium iodide).

In order to present the final data by fold expansion, the total number of cells and the absolute number of CD34+CD38- cells were calculated on a per milliliter basis (total number of cells = 1 mL × cell concentration; absolute number of CD34+CD38- cells = percentage of CD34+CD38- cells (%) × total number of cells).

(3) Results

As a result of the aforementioned study, expansion of CD34+CD38- cells was observed in all the truncated fragments other than H1-150 (FIG. 14). Since the analysis found that H30-208 and H30-240 had their activity, it was suggested that any peptide having the isoform-common (core) sequence (H30-240), in particular the region between positions 30 and 208, is capable of expressing its activity. Also, since no activity was observed in H1-150 lacking its nuclear localization sequence, it was considered that the nuclear localization sequence is essential for the expression of activity. From the above, it was suggested that hHDGFa peptides having the sequence of positions 30 to 208 have CD34+CD38- cell expansion activity, and this sequence was considered important for the expression of HDGF activity.

From the results given hereinabove, it was demonstrated that the HDGF substance, which is a factor inducing the expansion of hematopoietic stem cells, in particular, long-term hematopoietic stem cells, is capable of not only inducing and promoting in vitro expansion of hematopoietic stem cells but also inducing and promoting in vivo expansion of hematopoietic stem cells through in vivo administration, and therefore, it was confirmed that the hematopoietic stem cell expansion inducer of the present invention has a wide variety of utilities.

INDUSTRIAL APPLICABILITY

The HDGF substance of the present invention is capable of inducing expansion of hematopoietic stem cells and/or hematopoietic progenitor cells in vitro. The cells expanded or genetically transfected with the HDGF substance are expected to be usable as a cell material for transplantation therapy of diseases associated with impaired hematopoietic function or impaired immune function. Also, since the HDGF substance has activity to induce expansion of hematopoietic stem cells and/or hematopoietic progenitor cells, the HDGF substance is expected to be applicable as a pharmaceutical product for diseases associated with decreased hematopoietic stem cells and/or hematopoietic progenitor cells.

## Claims

1. An expansion inducer for hematopoietic stem cells, comprising at least one HDGF substance.

2. The expansion inducer according to claim 1, wherein the HDGF substance is selected from:

   (a) a polypeptide comprising the amino acid sequence of positions 30 to 208 of SEQ ID NO:1;
   (b) a polypeptide consisting of an amino acid sequence having at least 60% amino acid sequence similarity to the amino acid sequence of SEQ ID NO:1;
   (c) a polypeptide consisting of an amino acid sequence derived from the amino acid sequence of SEQ ID NO:1 by deletion, substitution and/or addition of one or several amino acids at one or several positions; or
   (d) a polypeptide comprising an amino acid sequence encoded by a nucleotide sequence capable of hybridizing under stringent conditions to a polynucleotide comprising a nucleotide sequence complementary to the nucleotide sequence of SEQ ID NO:2.

3. The expansion inducer according to claim 1, wherein the HDGF substance comprises a polypeptide consisting of the amino acid sequence of any of SEQ ID NOs:1 and 3 to 10.

**4.** The expansion inducer according to claim 1, wherein the HDGF substance is a polypeptide consisting of the amino acid sequence of any of SEQ ID NOs:1 and 3 to 10.

**5.** The expansion inducer according to any of claims 1 to 4, further comprising at least one cytokine selected from the group consisting of stem cell factors (SCF), flk-2/flt-3 ligand (FL), thrombopoietin (TPO), granulocyte-macrophage colony-stimulating factors (GM-CSF) and IL-6.

**6.** The expansion inducer according to claim 5, comprising at least thrombopoietin.

**7.** The expansion inducer according to any of claims 1 to 6, wherein the expansion inducer is administered to a subject for the purpose of treatment of a disease caused by depressed or impaired hematopoietic function.

**8.** The expansion inducer according to any of claims 1 to 6, wherein the expansion inducer is used to prepare a biological material for transplantation therapy in a subject with depressed or impaired hematopoietic function.

**9.** The expansion inducer according to claim 8, wherein the expansion inducer is used in a reagent, a culture medium, or a culture substrate.

**10.** A process for expanding hematopoietic stem cells, the process comprising the step of contacting the expansion inducer according to any of claims 1 to 6 in vitro with a sample comprising hematopoietic stem cells.

**11.** The process according to claim 10, wherein the sample comprising hematopoietic stem cells is a sample derived from the bone marrow, peripheral blood, or umbilical cord blood collected from a living organism.

**12.** A process for producing a biological material comprising hematopoietic stem cells, the process comprising the step of expanding hematopoietic stem cells in vitro using the process according to claim 10 or 11.

**13.** A biological material comprising hematopoietic stem cells, the biological material produced by the process according to claim 12.

**14.** The biological material according to claim 13, wherein the biological material is intended for use in transplantation therapy.

**15.** A process for producing a biological material comprising mature blood cells, the process comprising the steps of: contacting the expansion inducer according to any of claims 1 to 6 in vitro with a sample comprising hematopoietic stem cells to expand the hematopoietic stem cells; and inducing the expanded hematopoietic stem cells to differentiate into mature blood cells.

**16.** A biological material comprising mature blood cells, the biological material produced by the process according to claim 15.

**17.** The biological material according to claim 16, wherein the biological material is intended for use in transplantation medicine.

**18.** A process for producing a biological material comprising hematopoietic stem cells, the process comprising the steps of: contacting the expansion inducer according to any of claims 1 to 6 in vitro with a sample comprising hematopoietic stem cells to expand the hematopoietic stem cells; and introducing a gene for gene therapy into the hematopoietic stem cells before or after the contact with the expansion inducer.

**19.** The process according to claim 18, wherein the gene for gene therapy is selected from the group consisting of: the ADA gene for treatment of adenosine deaminase deficiency, the γc chain gene for treatment of X-linked severe combined immunodeficiency disease, the gp91-phox gene for treatment of chronic granulomatous disease, the glucocerebrosidase gene for treatment of Gaucher's disease, and a factor VIII or IX gene for treatment of hemophilia.

**20.** A biological material comprising hematopoietic stem cells, the biological material produced by the process according to claim 18 or 19.

# FIG. 1

**Protein sequence of hHDGFa**

msrsnrqkeykcgdlvfakmkgyphwparidempeaavkstankyqvfffgthetaflgpkdlfpyeeskekfgkpnkrk
gfseglweiennptvkasgyqssqkkscveepepepeaaegdgdkkgnaegssdeegklvidepakeknekgalkrrag
dlledspkrpkeaenpegeekeaatleverplpmeveknstpsepgsgrgppqeeeeeedeeeeatkedaeapgird
hesl

**Genome sequence of hHDGFa**

atgtcgcgatccaaccggcagaaggagtacaaatgcggggacctggtgttcgccaagatgaagggctacccacactggccgg
cccggattgacgagatgcctgaggctgccgtgaaatcaacagccaacaaataccaagtcttttttttcgggacccacgagacgg
cattcctgggccccaaagacctcttcccttacgaggaatccaaggagaagtttggcaagcccaacaagaggaaa
gggttcagcgaggggctgtgggagatcgagaacaaccctactgtcaaggcttccggctatcagtcctcccagaaaaagagctg
tgtggaagagcctgaaccagagcccgaagctgcagagggtgacggtgataagaaggggaatgcagagggcagcagcgacg
aggaagggaagctggtcattgatgagccagccaaggagaagaacgagaaaggagcgttgaagaggagagcaggggac
ttgctggaggactctcctaaacgtcccaaggaggcagaaaaccctgaaggagaggagaaggaggcagccaccttggaggttg
agaggccccttcctatggaggtggaaaagaatagcacccccctctgagcccggctctggccgggggcctccccaagaggaaga
agaagaggaggatgaagaggaagaggctaccaaggaagatgctgaggccccaggcatcagagatcatgagagcctg
tag

# FIG. 2

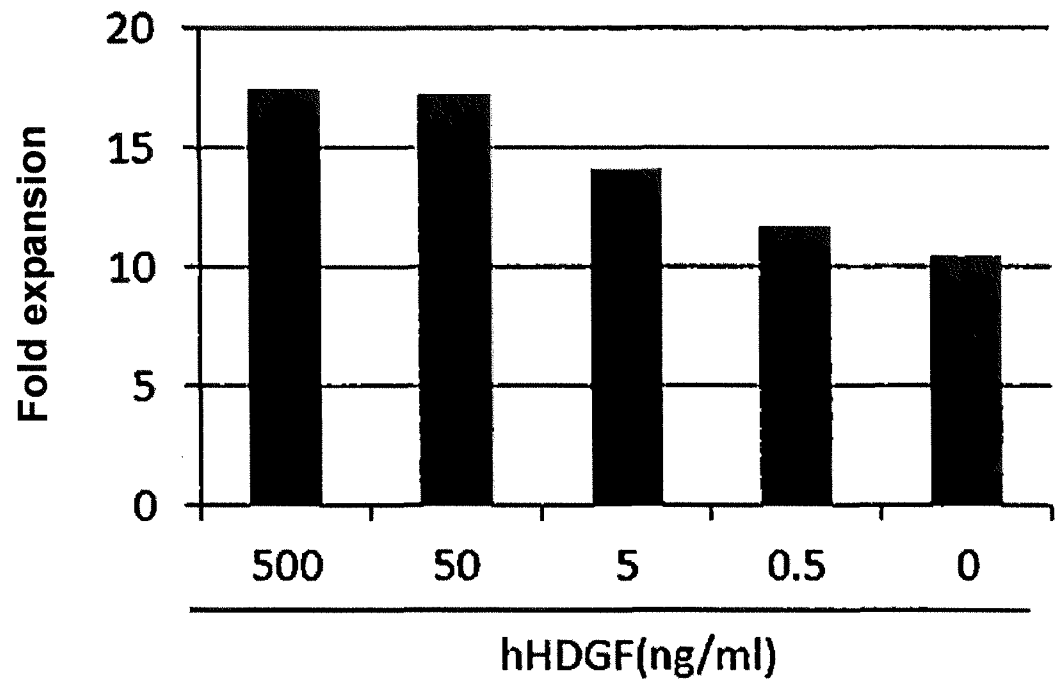

FIG. 3

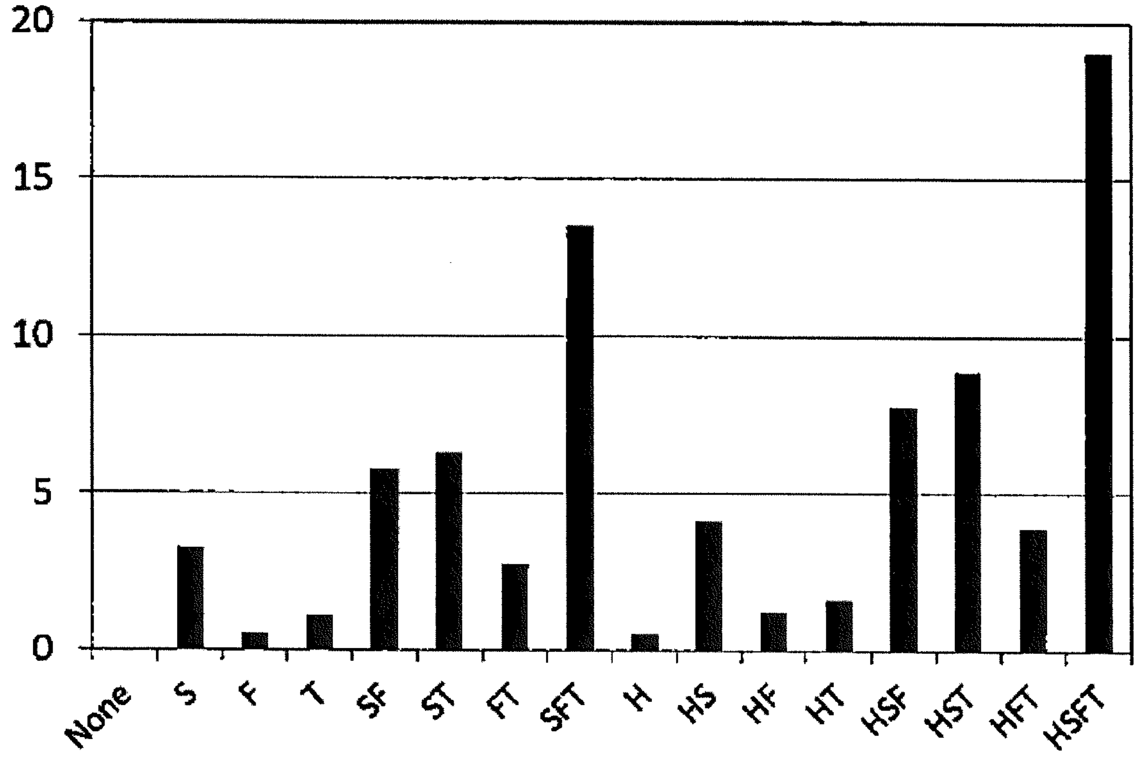

FIG. 4

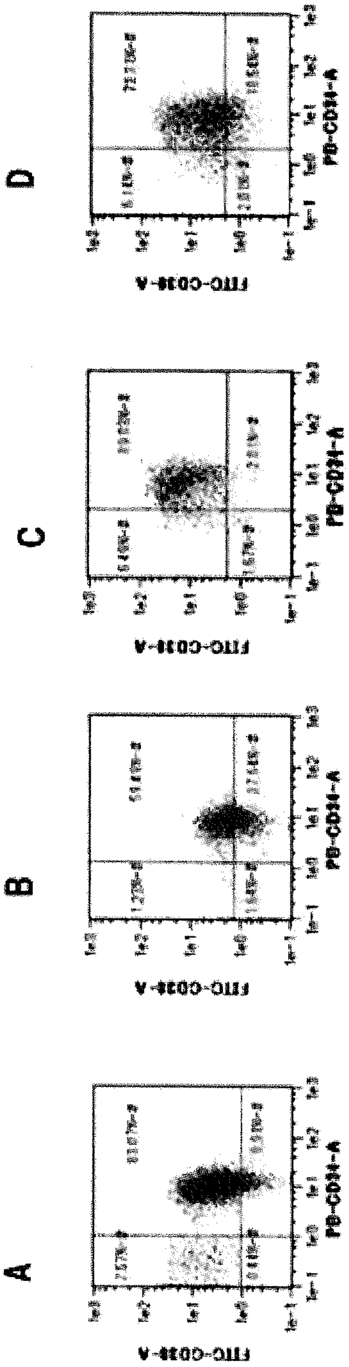

FIG. 5

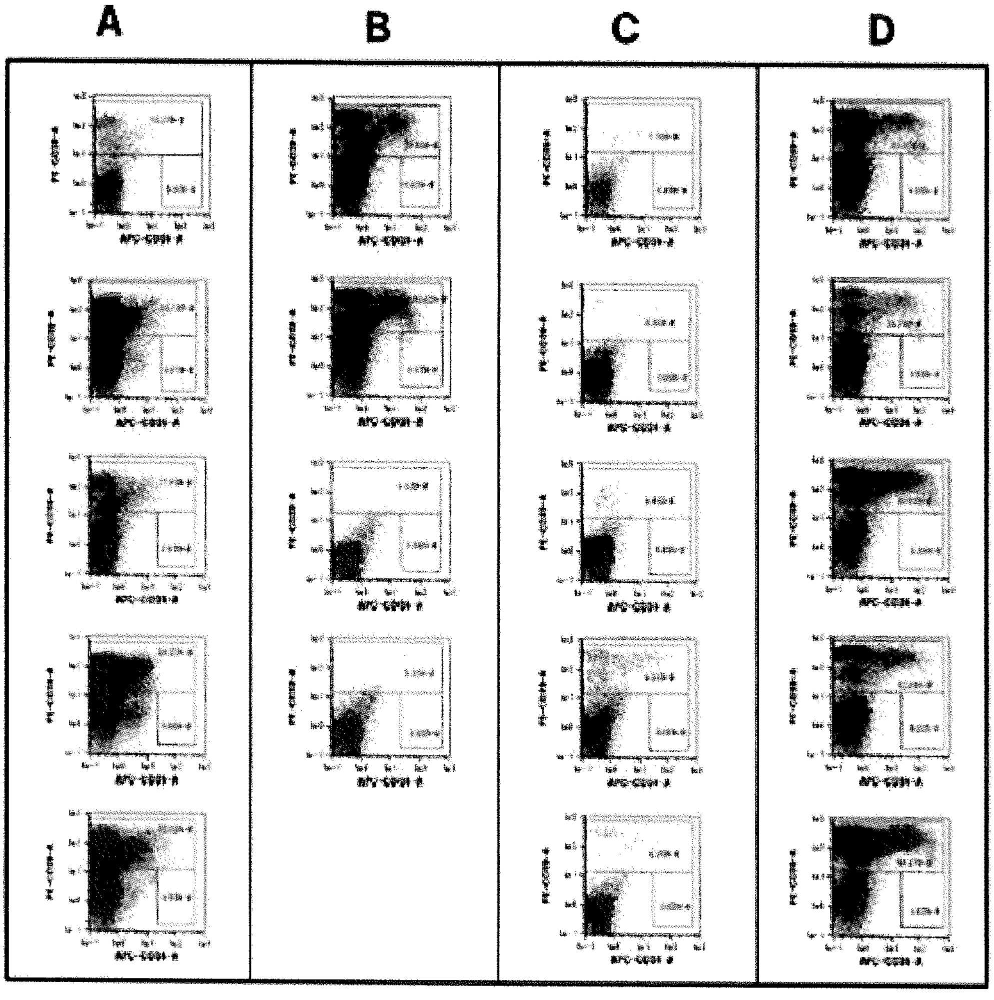

FIG. 6

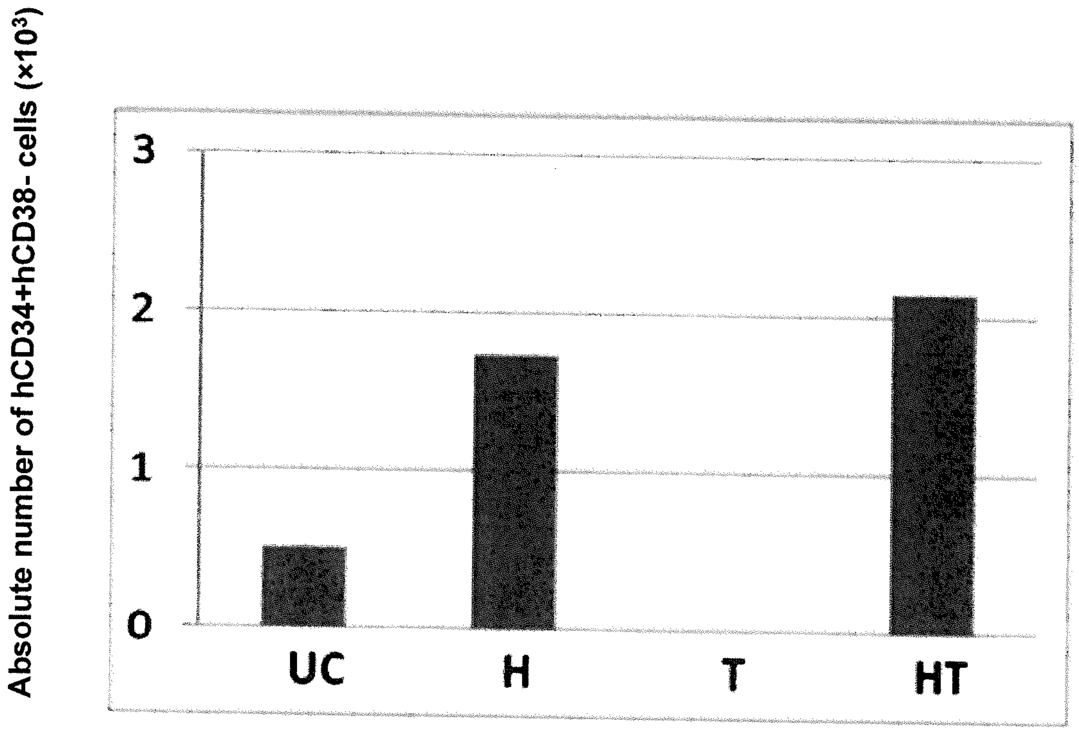
Absolute number of hCD34+hCD38- cells (×10³)
3
2
1
0
UC
H
T
HT

FIG. 7

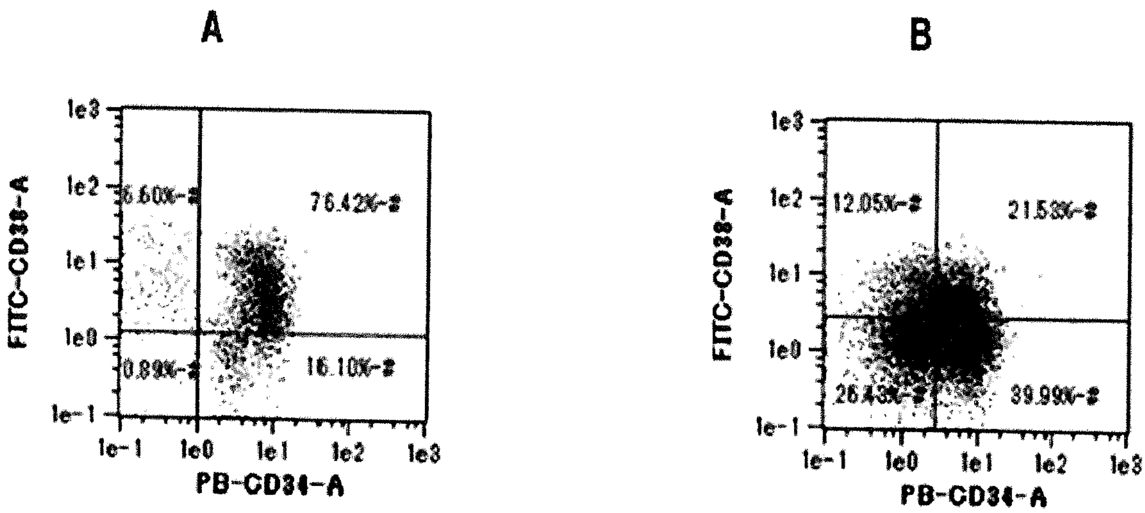
A
B
FITC-CD38-A
PB-CD34-A
8.60%-#
76.42%-#
0.89%-#
16.10%-#
12.05%-#
21.53%-#
26.43%-#
39.99%-#
1e-1
1e0
1e1
1e2
1e3

FIG. 8

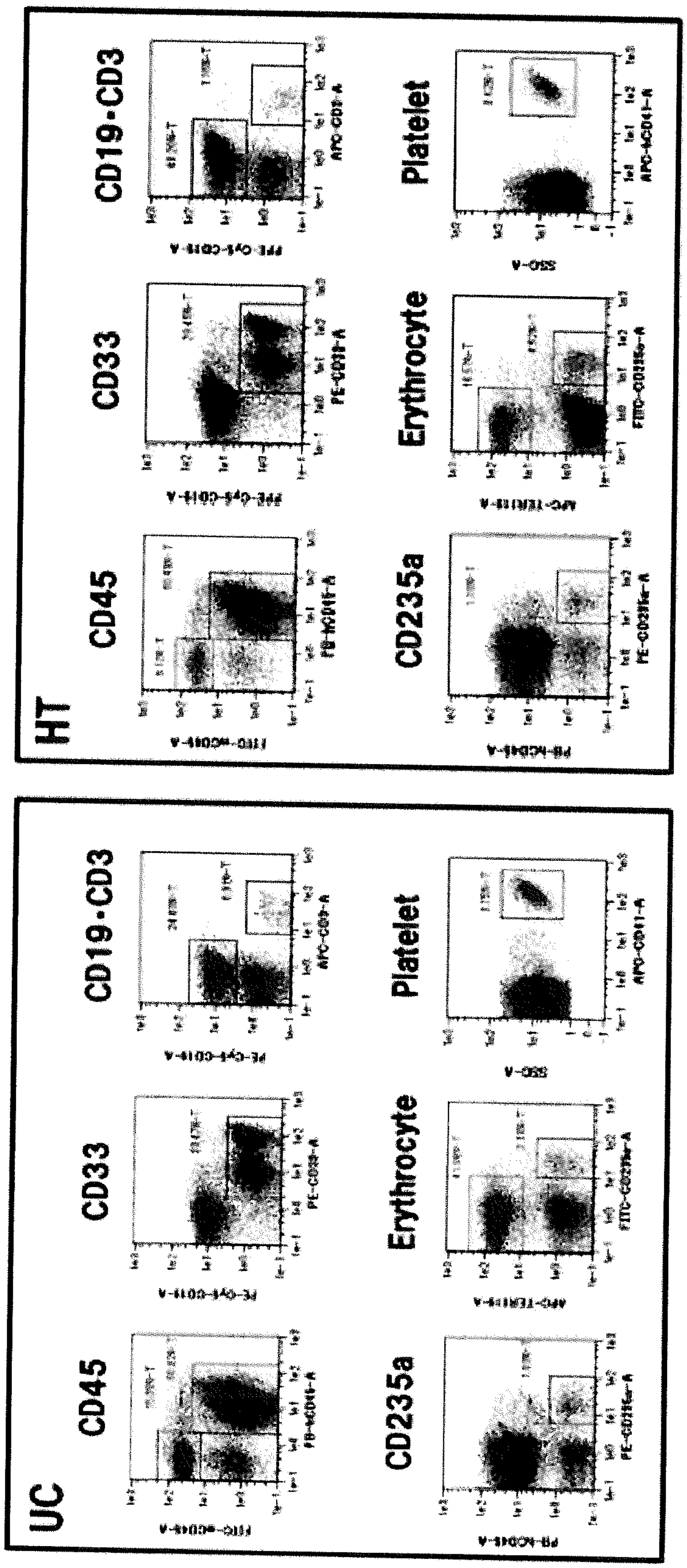
HT
CD45
CD33
CD19•CD3
CD235a
Erythrocyte
Platelet
UC
CD45
CD33
CD19•CD3
CD235a
Erythrocyte
Platelet

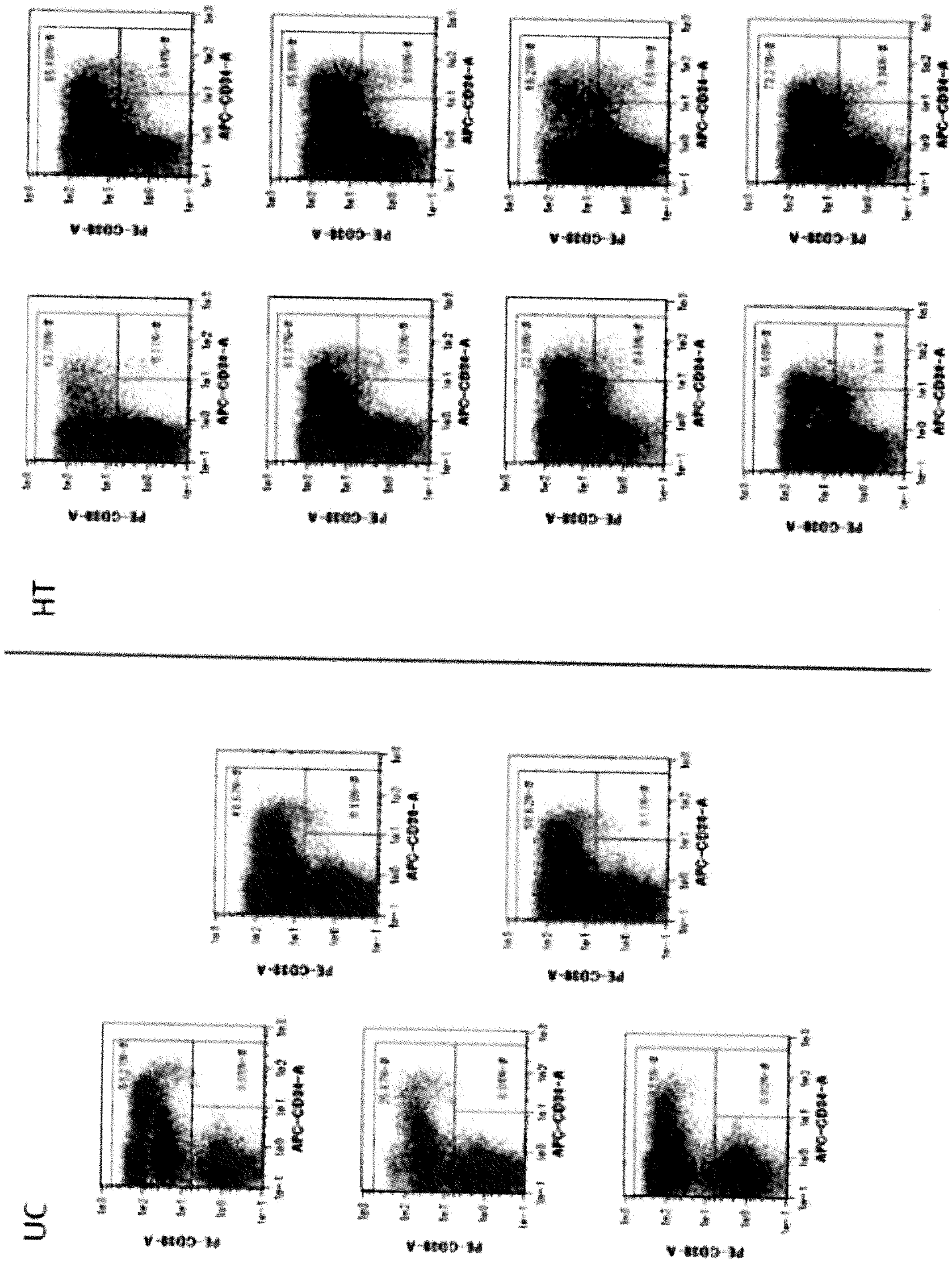


FIG. 9

FIG. 10

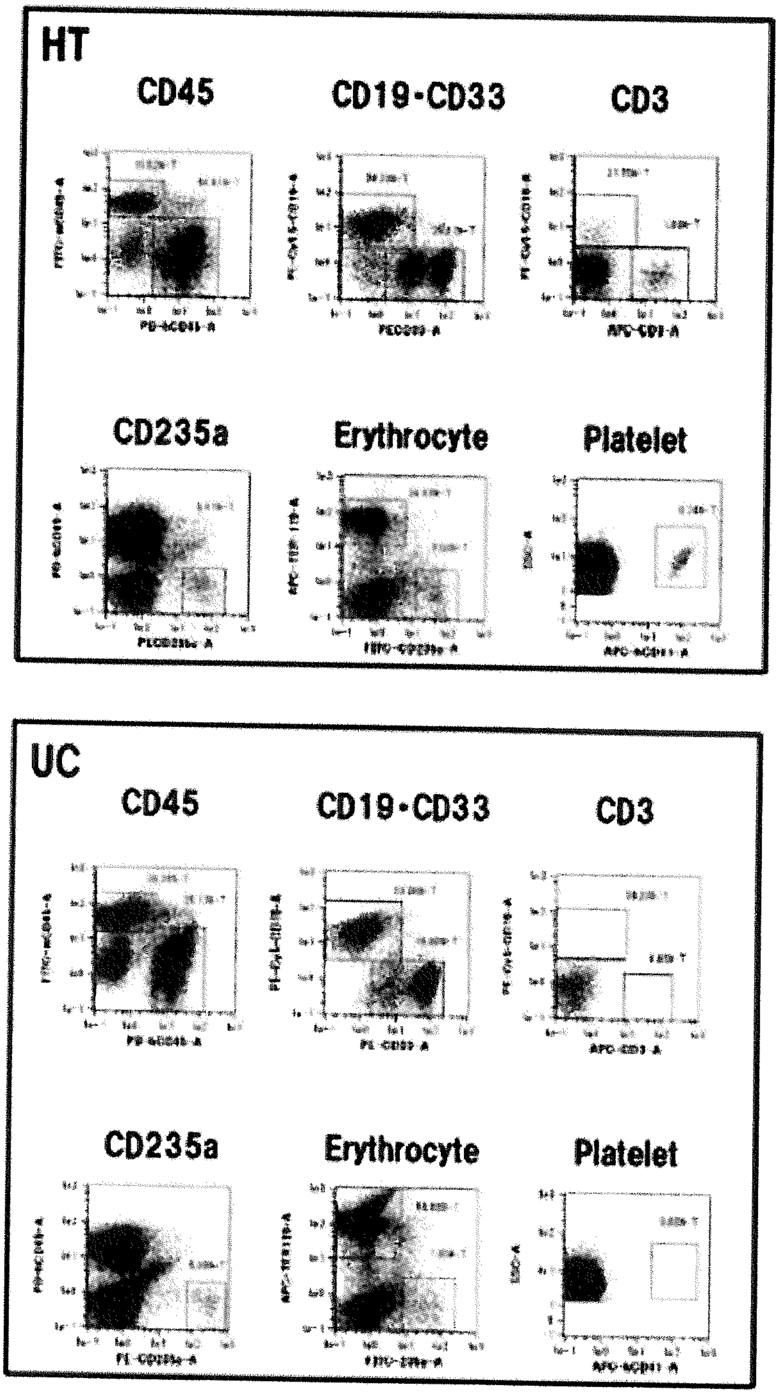
HT
CD45
CD19·CD33
CD3
CD235a
Erythrocyte
Platelet
UC
CD45
CD19·CD33
CD3
CD235a
Erythrocyte
Platelet

FIG. 11

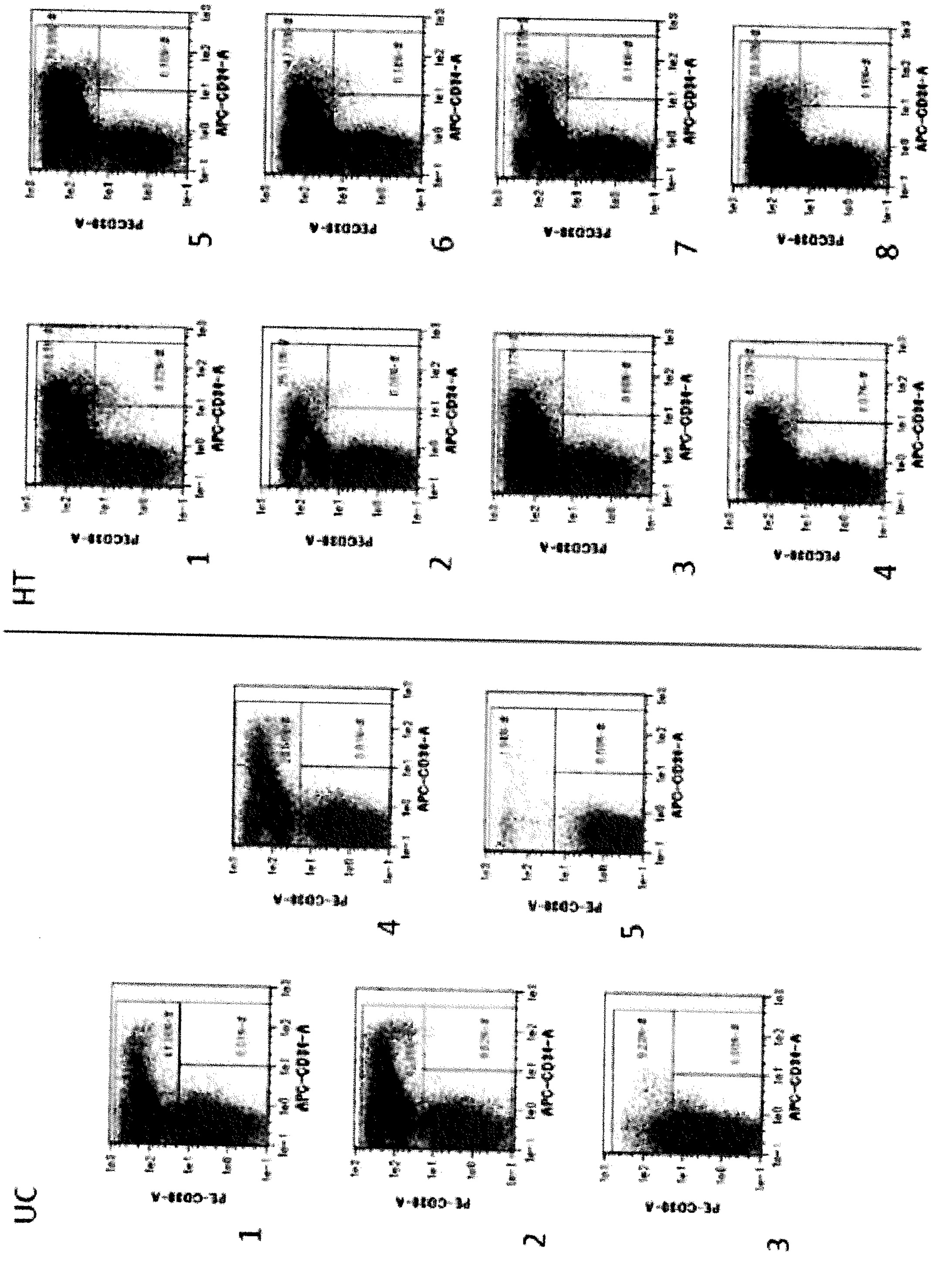

FIG. 12

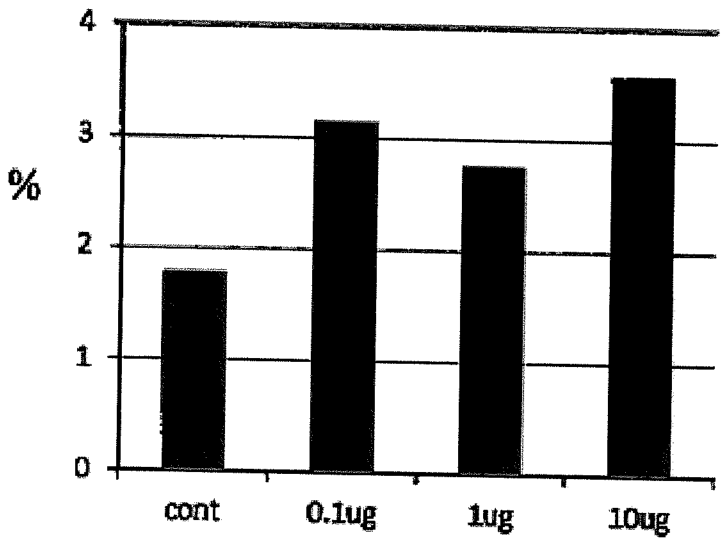
%
4
3
2
1
0
cont
0.1ug
1ug
10ug

FIG. 13

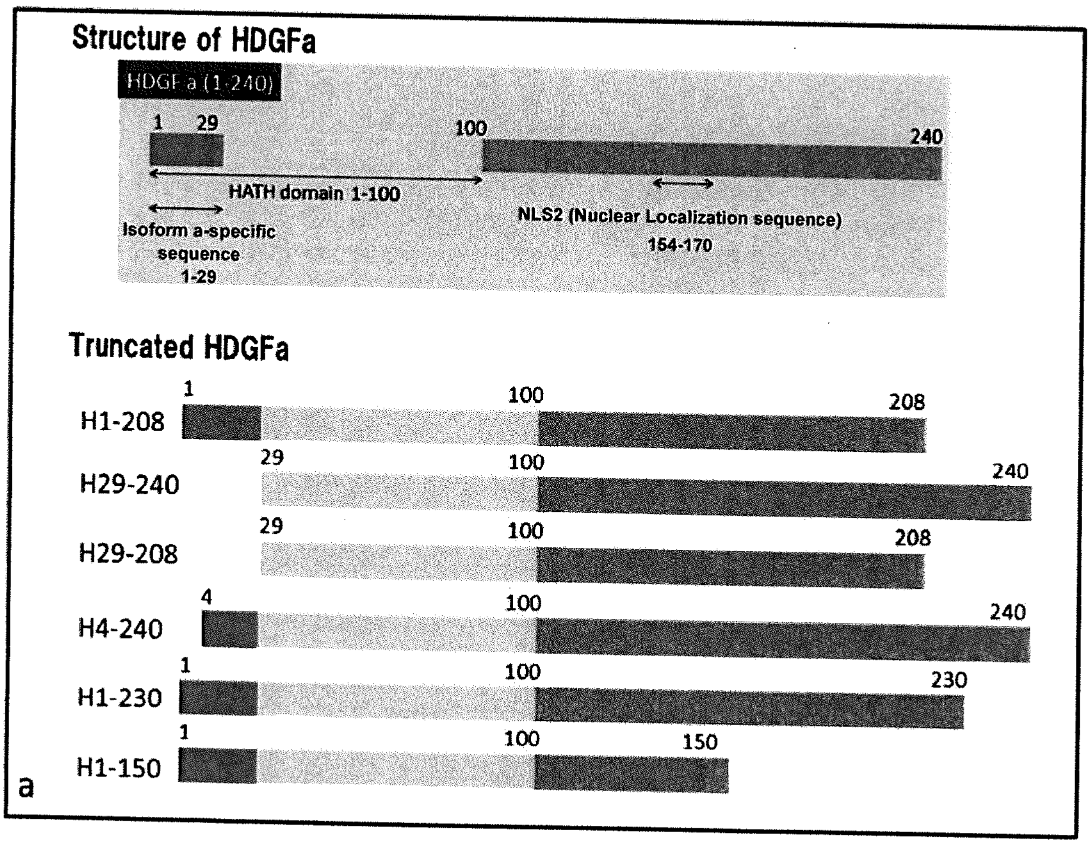
Structure of HDGFa
HDGF a (1-240)
1
29
100
240
HATH domain 1-100
Isoform a-specific sequence 1-29
NLS2 (Nuclear Localization sequence) 154-170
Truncated HDGFa
H1-208
H29-240
H29-208
H4-240
H1-230
H1-150
a

FIG. 14

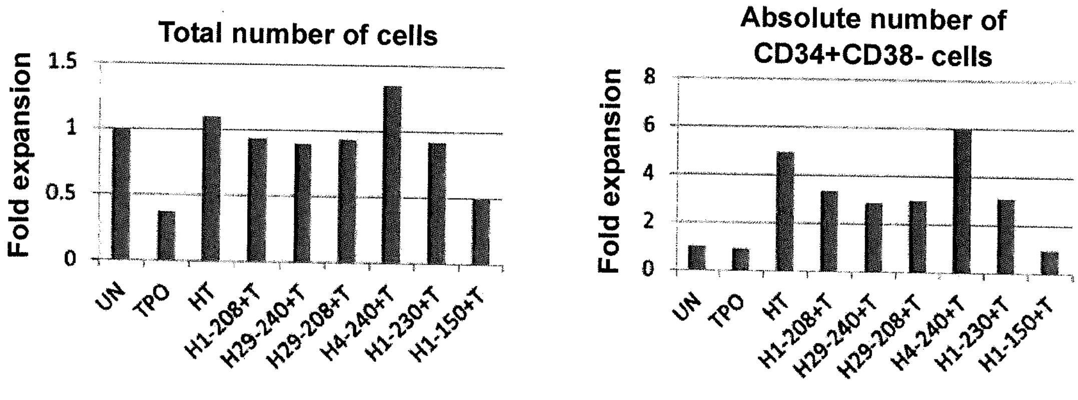
Total number of cells
Fold expansion
1.5
1
0.5
0
UN
TPO
HT
H1-208+T
H29-240+T
H29-208+T
H4-240+T
H1-230+T
H1-150+T
Absolute number of CD34+CD38- cells
Fold expansion
8
6
4
2
0
UN
TPO
HT
H1-208+T
H29-240+T
H29-208+T
H4-240+T
H1-230+T
H1-150+T

**INTERNATIONAL SEARCH REPORT**

International application No.
PCT/JP2016/061889

A. CLASSIFICATION OF SUBJECT MATTER
*A61K38/00*(2006.01)i, *A61K35/28*(2015.01)i, *A61P7/06*(2006.01)i, *A61P43/00*(2006.01)i, *C12N5/0789*(2010.01)i

According to International Patent Classification (IPC) or to both national classification and IPC

B. FIELDS SEARCHED

Minimum documentation searched (classification system followed by classification symbols)
A61K38/00, A61K35/28, A61P7/06, A61P43/00, C12N5/0789

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
JSTPlus/JMEDPlus/JST7580(JDreamIII), CAplus/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X | WO 2011/040500 A1 (Kyushu University), 07 April 2011 (07.04.2011), paragraphs [0108] to [0114], [0133] to [0151], [0157] to [0161] & US 2012/0315701 A1 paragraphs [0107] to [0113], [0107] to [0159], [0166] to [0170] & EP 2484689 A1 | 13,14,16,17, 20 |
| X | Akira NIWA et al., "Ex vivo expansion of human hematopoietic stem cells : past, present and beyond", Journal of Clinical and Experimental Medicine, 2012, vol.240, no.5, pages 471 to 476, ISSN:0039-2359 page 473, right column, line 12 to page 474, left column, line 29, fig. 5 | 13,14,16,17, 20 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 May 2016 (25.05.16) | 07 June 2016 (07.06.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2016/061889

C (Continuation). DOCUMENTS CONSIDERED TO BE RELEVANT

| Category* | Citation of document, with indication, where appropriate, of the relevant passages | Relevant to claim No. |
|---|---|---|
| A | GOMEZ E. et al., Hepatoma-derived growth factor: from the bovine uterus to the in vitro embryo culture., Reproduction, 2014, Vol.148, No.4, pages 353 to 365, ISSN:1470-1626 | 1-20 |
| A | ENOMOTO H. et al., Hepatoma-derived growth factor is highly expressed in developing liver and promotes fetal hepatocyte proliferation., Hepatology, Vol.36, No.6, 2002, pages 1519 to 1527, ISSN:0270-9139 | 1-20 |
| A | STEIN S.J. et al., Deletion of the NF-κB subunit p65/RelA in the hematopoietic compartment leads to defects in hematopoietic stem cell function., Blood, 2013, Vol.121, No.25, pages 5015 to 5024, ISSN:0006-4971 | 1-20 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **PELED T. ; GLUCKMAN E. ; HASSON N. ; ADI S. ; ASSOR H. ; YUDIN D. et al.** Chelatable cellular copper modulates differentiation and self-renewal of cord blood-derived hematopoietic progenitor cells. *Exp Hematol,* 2005, vol. 33, 1092-1100
- **BOITANO A.E. ; WANG J. ; ROMEO R. et al.** Aryl hydrocarbon receptor antagonists promote the expansion of human hematopoietic stem cells. *Science,* 2010, vol. 329 (5997), 1345-1348
- **AMSELLEM S. ; PFLUMIO F. ; BARDINET D. ; IZAC B. ; CHARNEAU P. ; ROMEO P.H. et al.** In vitro expansion of human hematopoietic stem cells by direct delivery of the HOXB4 homeoprotein. *Nat Med.,* 2003, vol. 9, 1423-7
- **NAKAMURA H. ; IZUMOTO Y. ; KAMBE H. ; KURODA T. ; MORI T. ; KAWAMURA K. ; YAMAMOTO H. ; KISHIMOTO T.** *J. Biol. Chem.,* 1994, vol. 269, 25143-25149
- **IZUMOTO Y. ; KURODA T. ; HARADA H. ; KISHIMOTO T. ; NAKAMURA H.** Hepatoma-derived growth factor belongs to a gene family in mice showing significant homology in the amino terminus. *Biochem Bioplays Res Commun.,* 1997, vol. 238 (1), 26-32
- **ENOMOTO H. ; YOSHIDA K. ; KISHIMA Y. ; KINOSHITA T. ; YAMAMOTO M. ; EVERETT A.D. ; MIYAJIMA A. ; NAKAMURA H.** Hepatoma-derived growth factor is highly expressed in developing liver and promotes fetal hepatocyte proliferation. *Hepatology,* 2002, vol. 36 (6), 1519-27
- *Blood,* 2012, vol. 100 (9), 113-1124
- **UEDA T. et al.** *J. Clin. Invest.,* 2000, vol. 105, 1013-1021
- **BLAESE R.M. et al.** *Science,* 1995, vol. 270 (5235), 475-480
- **HACEN-BAY-ABINA S. et al.** *N Engl J Med.,* 2002, vol. 346, 1185-1193
- *Experimental Medicine,* 1994, vol. 12, 303